# EUROPEAN PATENT SPECIFICATION

(11) **EP 1 799 817 B1**
(45) Date of publication and mention of the grant of the patent: **21.11.2012**
(21) Application number: 05801289.9
(22) Date of filing: 11.10.2005
(51) Int. Cl.: C12N 9/02, C12P 35/00, C12N 15/53

(54) **MUTANT EXPANDASES**
MODIFIZIERTE EXPANDASEN
EXPANDASES MUTANTES

(30) Priority: 13.10.2004 EP 04105022; 12.07.2005 EP 05106347
(43) Date of publication of application: 27.06.2007
(73) Proprietor: DSM Sinochem Pharmaceuticals Netherlands B.V., 2613 AX Delft (NL)
(72) Inventor: SCHIPPER, Dick, NL-2612 HL DELFT (NL); KERKMAN, Richard, NL-2042 NL ZANDVOORT (NL); RAAMSDONK, Lourina, Madeleine, NL-2496 LN DEN HAAG (NL); BOVENBERG, Roelof, Ary, Lans, NL-3062 DA ROTTERDAM (NL); JENNE, Stephane J., Foster City, California 94404 (US); CHEN, Yan, Santa Clara, California 95051 (US); CHEN, Yong Hong, Foster City, California 94404 (US); GAO, Xiaodong, Sunnyvale, California 94087 (US); KREBBER, Anke, Palo Alto, California 94303 (US); KREBBER, Claus, Palo Alto, California 94303 (US); TO LA CHING, Charlene, San Jose, California 95124 (US); TOBIN, Matthew, San Carlos, California 94070 (US)
(74) Representative: Misset, Onno
(86) International application number: PCT/EP2005/055148
(87) International publication number: WO 2006/040313

(56) References cited:
- WO-A1-95/04148
- DATABASE GENESEQ [Online] Derwent; 2 July 1998 (1998-07-02), BOVENBERG ET AL.: "S. clavuligerus expandase protein" XP002370227 Database accession no. AAW40424 & WO 98/02551 A (GIST-BROCADES) 22 January 1998 (1998-01-22)
- DATABASE GENESEQ [Online] Derwent; 17 February 1998 (1998-02-17), SUTHERLAND ET AL.: "Expandase mutant C155V" XP002370228 Database accession no. AAW34172 & WO 97/20053 A (GIST-BROCADES) 5 June 1997 (1997-06-05)
- DATABASE GENESEQ [Online] Derwent; 17 February 1998 (1998-02-17), SUTHERLAND ET AL.: "Expandase mutant R74F" XP002370229 Database accession no. AAW34166 & WO 97/20053 A (GIST-BROCADES) 5 June 1997 (1997-06-05)
- DATABASE GENESEQ [Online] Derwent; 12 March 2002 (2002-03-12), JOHNSON ET AL.: "S. clavuligerus penicillin N expandase 5 residue deletion at C-terminus" XP002370230 Database accession no. AAU11523 & WO 01/85951 A (ACS DOBFAR UK LTD.) 15 November 2001 (2001-11-15)
- DATABASE GENESEQ [Online] Derwent; 12 March 2002 (2002-03-12), JOHNSON ET AL.: "S. clavuligerus penicillin N expandase K310S deletion mutant" XP002370231 Database accession no. AAU11535 & WO 01/85951 A (ACS DOBFAR UK LTD.) 15 November 2001 (2001-11-15)

### SEQUENCE LISTING

<110> DSM IP Assets B.V.
<120> Mutant expandases
<130> 24172WO
<160> 14
<170> PatentIn version 3.1
<210> 1
   <211> 936
   <212> DNA
   <213> Streptomyces clavuligerus
<220>
   <221> exon
   <222> (1) .. (933)
   <223>
<400> 1
   atg gac acg acg gtg ccc acc ttc agc ctg gcc gaa ctc cag cag ggc      48
   Met Asp Thr Thr Val Pro Thr Phe Ser Leu Ala Glu Leu Gln Gln Gly
   1               5                   10                  15
   ctg cac cag gac gag ttc cgc agg tgt ctg agg gac aag ggc ctc ttc      96
   Leu His Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag     144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc     192
   Asp Ile Val Ile Asp Phe Phe Glu His Gly Ser Glu Ala Glu Lys Arg
       50                  55                  60
   gcc gtc acc tcg ccc gtc ccc acc atg cgc cgc ggc ttc acc ggg ctg     240
   Ala Val Thr Ser Pro Val Pro Thr Met Arg Arg Gly Phe Thr Gly Leu
   65                  70                  75                  80
   gag tcg gag agc acc gcc cag atc acc aat acc ggc agc tac tcc gac     288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc     336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc     384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag     432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg      480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag      528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc      576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc      624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac cat gtc gcg gcc ccc cgc agg gac cag ata gcg ggc agc      768
   Pro Arg His His Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc gat gtc agc ctg gac      864
   Phe Ser Val Pro Leu Ala Arg Glu Cys Gly Phe Asp Val Ser Leu Asp
           275                 280                 285
   ggc gag acc gcc acg ttc cag gat tgg atc ggg ggc aac tac gtg aac      912
   Gly Glu Thr Ala Thr Phe Gln Asp Trp Ile Gly Gly Asn Tyr Val Asn
       290                 295                 300
   atc cgc cgc aca tcc aag gca tga                                      936
   Ile Arg Arg Thr Ser Lys Ala
   305                 310
<210> 2
   <211> 970
   <212> DNA
   <213> Nocardia lactamdurans
<220>
   <221> exon
   <222> (1)..(942)
   <223>
<400> 2
   atg acg gac gcg acc gtg ccg acc ttc gat ctg gcc gag ctg cgt gag      48
   Met Thr Asp Ala Thr Val Pro Thr Phe Asp Leu Ala Glu Leu Arg Glu
   1               5                   10                  15
   ggc ttg cac cag gag gag ttc cgc cac tgc ctg cgc gag aag ggc gtg      96
   Gly Leu His Gln Glu Glu Phe Arg His Cys Leu Arg Glu Lys Gly Val
               20                  25                  30
   ttc tac ctc aag ggc acc ggg ctc gcc gag gcg gac cac gcc tcg gcg     144
   Phe Tyr Leu Lys Gly Thr Gly Leu Ala Glu Ala Asp His Ala Ser Ala
           35                  40                  45
   cgg gag atc gcg gtg gac ttc ttc gac cac ggc acc gag gcc gag aag     192
   Arg Glu Ile Ala Val Asp Phe Phe Asp His Gly Thr Glu Ala Glu Lys
       50                  55                  60
   aag gcg gtg atg acg ccg atc ccg acc atc cgg cgc ggg tac gcc ggg     240
   Lys Ala Val Met Thr Pro Ile Pro Thr Ile Arg Arg Gly Tyr Ala Gly
   65                  70                  75                  80
   ctg gag tcc gag agc acc gcg cag atc acg aac acc ggc aag tac acc     288
   Leu Glu Ser Glu Ser Thr Ala Gln Ile Thr Asn Thr Gly Lys Tyr Thr
                   85                  90                  95
   gac tac tcg atg tcg tac tcg atg ggc acc gcg gac aac ctg ttc ccc     336
   Asp Tyr Ser Met Ser Tyr Ser Met Gly Thr Ala Asp Asn Leu Phe Pro
               100                 105                 110
   agc gcc gag ttc gag aag gcg tgg gag gac tac ttc gcg cgg atg tac     384
   Ser Ala Glu Phe Glu Lys Ala Trp Glu Asp Tyr Phe Ala Arg Met Tyr
           115                 120                 125
   cgc gct tcg cag gac gtc gcg cgg cag gtg ctg acc tcg gtc ggc gcg     432
   Arg Ala Ser Gln Asp Val Ala Arg Gln Val Leu Thr Ser Val Gly Ala
       130                 135                 140
   gaa ccc gag gtc ggc atg gac gcc ttc ctc gac tgc gaa ccc ctg ctg     480
   Glu Pro Glu Val Gly Met Asp Ala Phe Leu Asp Cys Glu Pro Leu Leu
   145                 150                 155                 160
   cgc ctg cgc tac ttc ccc gag gtg ccc gag gat cgc gtg gcc gag gag     528
   Arg Leu Arg Tyr Phe Pro Glu Val Pro Glu Asp Arg Val Ala Glu Glu
                   165                 170                 175
   cag ccg ctg cgg atg gcc ccg cat tac gac ctc tcg atc gtc acc ctg     576
   Gln Pro Leu Arg Met Ala Pro His Tyr Asp Leu Ser Ile Val Thr Leu
               180                 185                 190
   atc cac cag acc cct tgc gcg aac ggg ttc gtc agc ctg cag gtc gag     624
   Ile His Gln Thr Pro Cys Ala Asn Gly Phe Val Ser Leu Gln Val Glu
           195                 200                 205
   gtg gac ggg tcc tat gtg gac atc ccg gcg cag ccg ggc gcg gtg ctg     672
   Val Asp Gly Ser Tyr Val Asp Ile Pro Ala Gln Pro Gly Ala Val Leu
       210                 215                 220
   gtg ttc tgc ggc gcg gtg gcg acg ctg gtg gcc gac ggc gcg atc aag      720
   Val Phe Cys Gly Ala Val Ala Thr Leu Val Ala Asp Gly Ala Ile Lys
   225                 230                 235                 240
   gcg ccc aag cac cac gtg gcc gcg ccc ggc gcg gac aag cgg gtg ggc      768
   Ala Pro Lys His His Val Ala Ala Pro Gly Ala Asp Lys Arg Val Gly
                   245                 250                 255
   agc agc cgc acc tcc agc gtg ttc ttc ctg cgc ccc aac ggg gac ttc      816
   Ser Ser Arg Thr Ser Ser Val Phe Phe Leu Arg Pro Asn Gly Asp Phe
               260                 265                 270
   cgc ttc tcg gtg ccg cgg gcc agg gag tgc ggg ttc gac gtc agc atc      864
   Arg Phe Ser Val Pro Arg Ala Arg Glu Cys Gly Phe Asp Val Ser Ile
           275                 280                 285
   ccg gcc gag acc gcc acc ttc gac gac tgg atc ggc ggc aac tac atc      912
   Pro Ala Glu Thr Ala Thr Phe Asp Asp Trp Ile Gly Gly Asn Tyr Ile
       290                 295                 300
   aac atc cgg aag acc gcc gcc gcc cgg ctt tgaaggctct tcatgacgag        962
   Asn Ile Arg Lys Thr Ala Ala Ala Arg Leu
   305                 310
   ccaatgca                                                             970
<210> 3
   <211> 936
   <212> DNA
   <213> Artificial
<220>
   <223> 779
<220>
   <221> exon
   <222> (1)..(936)
   <223>
<400> 3
   atg gac acg acg gtg ccc acc ttc agc ctg gcc gaa ctc cag cag ggc       48
   Met Asp Thr Thr Val Pro Thr Phe Ser Leu Ala Glu Leu Gln Gln Gly
   1               5                   10                  15
   ctg cgc cag gac gag ttc cgc agg tgt ctg agg gac aag ggc ctc ttc       96
   Leu Arg Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag      144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc      192
   Asp Ile Val Ile Asp Phe Phe Glu His Gly Ser Glu Ala Glu Lys Arg
       50                  55                  60
   gcc gtc acc tcg ccc gtc ccc acc atg cgc cgc ggc ttc acc ggg ctg      240
   Ala Val Thr Ser Pro Val Pro Thr Met Arg Arg Gly Phe Thr Gly Leu
   65                  70                  75                  80
   gag tcg gag agc acc gcc cag att acc aat acc ggc agc tac tcc gac      288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc      336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc      384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag      432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg      480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag      528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc      576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc      624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac cat gtc gcg gcc ccc cgc agg gac cag ata gcg ggc agc      768
   Pro Arg His His Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc aat gtc agc ctg gac      864
   Phe Ser Val Pro Leu Ala Arg Glu Cys Gly Phe Asn Val Ser Leu Asp
               275                 280                 285
   ggc gag acc gcc acg ttc cag gat tgg atc ggg gtc aac tac gtg aac      912
   Gly Glu Thr Ala Thr Phe Gln Asp Trp Ile Gly Val Asn Tyr Val Asn
       290                 295                 300
   atc cgc cgc aca tcc aag gca tga                                      936
   Ile Arg Arg Thr Ser Lys Ala
   305                 310
<210> 4
   <211> 939
   <212> DNA
   <213> Artificial sequence
<220>
   <223> H262
<220>
   <221> exon
   <222> (1)..(936)
   <223>
<400> 4
   atg acg gac gcg acc gtg ccg acc ttc gat ctg gcc gag ctg cgt gag       48
   Met Thr Asp Ala Thr Val Pro Thr Phe Asp Leu Ala Glu Leu Arg Glu
   1               5                   10                  15
   ggc ttg cac cag gag gag ttc cgc cac tgc ctg cgc gag aag ggc gtg       96
   Gly Leu His Gln Glu Glu Phe Arg His Cys Leu Arg Glu Lys Gly Val
               20                  25                  30
   ttc tac ctc aag ggc acc ggg ctc gcc gag gcg gac cac gcc tcg gcg      144
   Phe Tyr Leu Lys Gly Thr Gly Leu Ala Glu Ala Asp His Ala Ser Ala
           35                  40                  45
   cgg gag atc gcg gtg gac ttc ttc gag cac ggc agc gag gcg gag aag      192
   Arg Glu Ile Ala Val Asp Phe Phe Glu His Gly Ser Glu Ala Glu Lys
       50                  55                  60
   cgc gcc gtc acc tcg ccc gtc ccc acc atg cgc cgc ggc ttc acc ggg      240
   Arg Ala Val Thr Ser Pro Val Pro Thr Met Arg Arg Gly Phe Thr Gly
   65                  70                  75                  80
   ctg gag tcg gag agc acc gcg cag atc acg aac acc ggc agc tac tcc      288
   Leu Glu Ser Glu Ser Thr Ala Gln Ile Thr Asn Thr Gly Ser Tyr Ser
                   85                  90                  95
   gac tac tcg atg tgc tac tcg atg ggc acc gcg gac aac ctc ttc ccg      336
   Asp Tyr Ser Met Cys Tyr Ser Met Gly Thr Ala Asp Asn Leu Phe Pro
               100                 105                 110
   tcc ggt gac ttc gag cgg atc tgg acc cag tac ttc gac cgc cag tac      384
   Ser Gly Asp Phe Glu Arg Ile Trp Thr Gln Tyr Phe Asp Arg Gln Tyr
           115                 120                 125
   acc gcc tcc cgc gcg gtc gcc cgg gag gtc ctg cgg gcg acc ggg acc      432
   Thr Ala Ser Arg Ala Val Ala Arg Glu Val Leu Arg Ala Thr Gly Thr
       130                 135                 140
   gag ccc gac ggc ggg gtc gag gcc ttc ctc gac tgc gag ccg ctg ctg      480
   Glu Pro Asp Gly Gly Val Glu Ala Phe Leu Asp Cys Glu Pro Leu Leu
   145                 150                 155                 160
   cgg ttc cgc tac ttc ccg cag gtg ccc gag gat cgc gtg gcc gag gag      528
   Arg Phe Arg Tyr Phe Pro Gln Val Pro Glu Asp Arg Val Ala Glu Glu
                   165                 170                 175
   cag ccg ctg cgg atg gcc ccg cac tac gac ctc tcg atc gtc acc ctg      576
   Gln Pro Leu Arg Met Ala Pro His Tyr Asp Leu Ser Ile Val Thr Leu
               180                 185                 190
   atc cag cag aca ccc tgc gcc aac ggc ttc gtc agc ctc cag gcc gag      624
   Ile Gln Gln Thr Pro Cys Ala Asn Gly Phe Val Ser Leu Gln Ala Glu
           195                 200                 205
   gtc ggc ggc gcg ttc acg gac ctg ccc tac cgt ccg gac gcc gtc ctc      672
   Val Gly Gly Ala Phe Thr Asp Leu Pro Tyr Arg Pro Asp Ala Val Leu
       210                 215                 220
   gtc ttc tgc ggc gcc atc gcg acc ctg gtg acc ggc ggc cag gtc aag      720
   Val Phe Cys Gly Ala Ile Ala Thr Leu Val Thr Gly Gly Gln Val Lys
   225                 230                 235                 240
   gcc ccc cgg cac cat gtc gcg gcc ccc cgc agg gac cag ata gcg ggc      768
   Ala Pro Arg His His Val Ala Ala Pro Arg Arg Asp Gln Ile Ala Gly
                   245                 250                 255
   agc agc cgc acc tcc agc gtg ttc ttc ctg cgc ccc aac ggg gac ttc      816
   Ser Ser Arg Thr Ser Ser Val Phe Phe Leu Arg Pro Asn Gly Asp Phe
               260                 265                 270
   cgc ttc tcg gtg ccg cgg gcc agg gag tgc ggg ttc gac gtc ggc ctg      864
   Arg Phe Ser Val Pro Arg Ala Arg Glu Cys Gly Phe Asp Val Gly Leu
           275                 280                 285
   gac ggc gag acc gcc acg ttc cgg gat tgg atc ggg ggc aac tac gtg      912
   Asp Gly Glu Thr Ala Thr Phe Arg Asp Trp Ile Gly Gly Asn Tyr Val
       290                 295                 300
   aac atc cgc cgc aca tcc aag gca tag                                  939
   Asn Ile Arg Arg Thr Ser Lys Ala
   305                 310
<210> 5
   <211> 936
   <212> DNA
   <213> Artificial sequence
<220>
   <223> H127
<220>
   <221> exon
   <222> (1)..(933)
   <223>
<400> 5
   atg gac acg acg gtg ccc acc ttc agc ctg gcc gaa ctc cag cag ggc      48
   Met Asp Thr Thr Val Pro Thr Phe Ser Leu Ala Glu Leu Gln Gln Gly
   1               5                   10                  15
   ctg cgc cag gac gag ttc cgc agg tgt ctg agg gac aag ggc ctc ttc      96
   Leu Arg Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag     144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc     192
   Asp Ile Val Ile Asp Phe Phe Glu His Gly Ser Glu Ala Glu Lys Arg
       50                  55                  60
   gcc gtc acc tcg ccc gtc ccc acc atg cgc cgc ggc ttc acc ggg ctg     240
   Ala Val Thr Ser Pro Val Pro Thr Met Arg Arg Gly Phe Thr Gly Leu
   65                  70                  75                  80
   gag tcg gag agc acc gcc cag att acc aat acc ggc agc tac tcc gac     288
   Glu Ser Glu Ser Thr Ala Gln Ile Thr Asn Thr Gly Ser Tyr Ser Asp
                   85                  90                  95
   tac tcg atg tcg tac tcg atg ggc acc gcg gac aac ctg ttc ccc agc     336
   Tyr Ser Met Ser Tyr Ser Met Gly Thr Ala Asp Asn Leu Phe Pro Ser
               100                 105                 110
   gcc gag ttc gag aag gcg tgg gag gac tac ttc gcg cgg atg tac cgc     384
   Ala Glu Phe Glu Lys Ala Trp Glu Asp Tyr Phe Ala Arg Met Tyr Arg
           115                 120                 125
   gct tcg cag gac gtc gcg cgg cag gtg ctg acc tcg gtc ggc gcg gaa     432
   Ala Ser Gln Asp Val Ala Arg Gln Val Leu Thr Ser Val Gly Ala Glu
       130                 135                 140
   ccc gag gtc ggc atg gac gcc ttc ctc gac tgc gaa ccc ctg ctg cgc     480
   Pro Glu Val Gly Met Asp Ala Phe Leu Asp Cys Glu Pro Leu Leu Arg
   145                 150                 155                 160
   ctg cgc tac ttc ccc gag gtg ccc gag gat cgc gtg gcc gag gag cag     528
   Leu Arg Tyr Phe Pro Glu Val Pro Glu Asp Arg Val Ala Glu Glu Gln
                   165                 170                 175
   ccg ctg cgg atg gcc ccg cac tac gac ctg tcg atg gtc acc ctc atc     576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc     624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac cat gtc gcg gcc ccc cgc agg gac cag ata gcg ggc agc      768
   Pro Arg His His Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc aat gtc agc ctg gac      864
   Phe Ser Val Pro Leu Ala Arg Glu Cys Gly Phe Asn Val Ser Leu Asp
           275                 280                 285
   ggc gag acc gcc acg ttc cag gat tgg atc ggg gtc aac tac gtg aac      912
   Gly Glu Thr Ala Thr Phe Gln Asp Trp Ile Gly Val Asn Tyr Val Asn
       290                 295                 300
   atc cgc cgc aca tcc aag gca tga                                      936
   Ile Arg Arg Thr Ser Lys Ala
   305                 310
<210> 6
   <211> 962
   <212> DNA
   <213> Artificial sequence
<220>
   <223> H101
<220>
   <221> exon
   <222> (1)..(933)
   <223>
<400> 6
   atg gac acg acg gtg ccc acc ttc agc ctg gcc gaa ctc cag cag ggc       48
   Met Asp Thr Thr Val Pro Thr Phe Ser Leu Ala Glu Leu Gln Gln Gly
   1               5                   10                  15
   ctg cgc cag gac gag ttc cgc agg tgt ctg agg gac aag ggc ctc ttc       96
   Leu Arg Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag      144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc      192
   Asp Ile Val Ile Asp Phe Phe Glu His Gly Ser Glu Ala Glu Lys Arg
       50                  55                  60
   gcc gtc acc tcg ccc gtc ccc acc atg cgc cgc ggc ttc acc ggg ctg      240
   Ala Val Thr Ser Pro Val Pro Thr Met Arg Arg Gly Phe Thr Gly Leu
   65                  70                  75                  80
   gag tcg gag agc acc gcc cag att acc aat acc ggc agc tac tcc gac      288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc      336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc      384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag      432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg      480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag      528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc      576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc      624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac cat gtc gcg gcc ccc cgc agg gac cag ata gcg ggc agc      768
   Pro Arg His His Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc aat gtc agc ctg gac       864
   Phe Ser Val Pro Leu Ala Arg Glu Cys Gly Phe Asn Val Ser Leu Asp
           275                 280                 285
   ggc gag acc gcc acg ttc cag gat tgg atc ggg gtc aac tac gtg aac       912
   Gly Glu Thr Ala Thr Phe Gln Asp Trp Ile Gly Val Asn Tyr Val Asn
       290                 295                 300
   atc cgc cgc aca tcc aag gca tgataggctc ttcatgacga gaatatgca          962
   Ile Arg Arg Thr Ser Lys Ala
   305                 310
<210> 7
   <211> 936
   <212> DNA
   <213> Artificial sequence
<220>
   <223> H111
<220>
   <221> exon
   <222> (1)..(933)
   <223>
<400> 7
   atg gac acg acg gtg ccc acc ttc agc ctg gcc gaa ctc cag cag ggc        48
   Met Asp Thr Thr Val Pro Thr Phe Ser Leu Ala Glu Leu Gln Gln Gly
   1               5                   10                  15
   ctg cgc cag gac gag ttc cgc agg tgt ctg agg gac aag ggc ctc ttc        96
   Leu Arg Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag       144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc       192
   Asp Ile Val Ile Asp Phe Phe Glu His Gly Ser Glu Ala Glu Lys Arg
       50                  55                  60
   gcc gtc acc tcg ccc gtc ccc acc atg cgc cgc ggc ttc acc ggg ctg       240
   Ala Val Thr Ser Pro Val Pro Thr Met Arg Arg Gly Phe Thr Gly Leu
   65                  70                  75                  80
   gag tcg gag agc acc gcc cag att acc aat acc ggc agc tac tcc gac       288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc       336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc      384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag      432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg      480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag      528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc      576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc      624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac caa gtc gcg gcc ccc cgc agg gac cag ata gcg ggc agc      768
   Pro Arg His Gln Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc aat gtc agc ctg gac      864
   Phe Ser Val Pro Leu Ala Arg Glu Cys Gly Phe Asn Val Ser Leu Asp
           275                 280                 285
   ggc gag acc gcc acg ttc cag gat tgg atc ggg gtc aac tac gtg aac      912
   Gly Glu Thr Ala Thr Phe Gln Asp Trp Ile Gly Val Asn Tyr Val Asn
       290                 295                 300
   atc cgc cgc aca tcc aag gca tga                                      936
   Ile Arg Arg Thr Ser Lys Ala
   305                 310
<210> 8
   <211> 936
   <212> DNA
   <213> Artificial sequence
<220>
   <223> H122
<220>
   <221> exon
   <222> (1)..(933)
   <223>
<400> 8
   atg gac acg acg gtg ccc acc ttc agc ctg gcc gaa ctc cag cag ggc        48
   Met Asp Thr Thr Val Pro Thr Phe Ser Leu Ala Glu Leu Gln Gln Gly
   1               5                   10                  15
   ctg cac cag gac gag ttc cgc agg tgt ctg agg gac aag ggc ctc ttc        96
   Leu His Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag       144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc       192
   Asp Ile Val Ile Asp Phe Phe Glu His Gly Ser Glu Ala Glu Lys Arg
       50                  55                  60
   gcc gtc acc tcg ccc gtc ccc acc atg cgc cgc ggc ttc acc ggg ctg       240
   Ala Val Thr Ser Pro Val Pro Thr Met Arg Arg Gly Phe Thr Gly Leu
   65                  70                  75                  80
   gag tcg gag agc acc gcc cag atc acc aat acc ggc agc tac tcc gac       288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc       336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc       384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag       432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg       480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag       528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc       576
   Pro Leu Arg Met Ala Pro His Tyr Asp Leu Ser Met Val Thr Leu Ile
   180 185 190
   cag cag aca ccc tgc gcc aac ggc ttc gtc agc ctc cag gcc gag gtc 624
   Gln Gln Thr Pro Cys Ala Asn Gly Phe Val Ser Leu Gln Ala Glu Val
   195 200 205
   ggc ggc gcg ttc acg gac ctg ccc tac cgt ccg gac gcc gtc ctc gtc 672
   Gly Gly Ala Phe Thr Asp Leu Pro Tyr Arg Pro Asp Ala Val Leu Val
   210 215 220
   ttc tgc ggc gcc atc gcg acc ctg gtg acc ggc ggc cag gtc aag gcc 720
   Phe Cys Gly Ala Ile Ala Thr Leu Val Thr Gly Gly Gln Val Lys Ala
   225 230 235 240
   ccc cgg cac cat gtc gcg gcc ccc cgc agg gac cag ata gcg ggc agc 768
   Pro Arg His His Val Ala Ala Pro Arg Arg Asp Gln Ile Ala Gly Ser
   245 250 255
   agc cgc acc tcc agt gtg ttc ttc ctc cgt ccc aac gcg gac ttc acc 816
   Ser Arg Thr Ser Ser Val Phe Phe Leu Arg Pro Asn Ala Asp Phe Thr
   260 265 270
   ttc tcc gtc ccg ctg gcg cgc gag tgc ggc ttc aat gtc agc ctg gac 864
   Phe Ser Val Pro Leu Ala Arg Glu Cys Gly Phe Asn Val Ser Leu Asp
   275 280 285
   ggc gag acc gcc acg ttc cag gat tgg atc ggg gtc aac tac gtg aac 912
   Gly Glu Thr Ala Thr Phe Gln Asp Trp Ile Gly Val Asn Tyr Val Asn
   290 295 300
   atc cgc cgc aca tcc aag gca tga 936
   Ile Arg Arg Thr Ser Lys Ala
   305 310
<210> 9
   <211> 936
   <212> DNA
   <213> Artificial sequence
<220>
   <223> H301
<220>
   <221> exon
   <222> (1)..(933)
   <223>
<400> 9
   atg gac acg acg gtg ccc acc ttc agc ctg gcc gaa ctc cag cag ggc 48
   Met Asp Thr Thr Val Pro Thr Phe Ser Leu Ala Glu Leu Gln Gln Gly
   1 5 10 15
   ctg cgc cag gac gag ttc cgc agg tgt ctg agg gac aag ggc ctc ttc 96
   Leu Arg Gln Asp Glu Phe Arg Arg Cys Leu Arg Asp Lys Gly Leu Phe
   20 25 30
   tat ctg acg gac tgc ggt ctg acc gac acc gag ctg aag tcg gcc aag      144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc      192
   Asp Ile Val Ile Asp Phe Phe Glu His Gly Ser Glu Ala Glu Lys Arg
       50                  55                  60
   gcc gtc acc tcg ccc gtc ccc acc acg cgc cgc ggc ttc acc ggg ctg      240
   Ala Val Thr Ser Pro Val Pro Thr Thr Arg Arg Gly Phe Thr Gly Leu
   65                  70                  75                  80
   gag tcg gag agc acc gcc cag att acc aat acc ggc agc tac tcc gac      288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc      336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc      384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag      432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg      480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag      528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc      576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc      624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac cat gtc gcg gcc ccc cgc agg gac cag ata gcg ggc agc      768
   Pro Arg His His Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc aat gtc agc ctg gac      864
   Phe Ser Val Pro Leu Ala Arg Glu Cys Gly Phe Asn Val Ser Leu Asp
           275                 280                 285
   ggc gag acc gcc acg ttc cag gat tgg atc ggg gtc aac tac gtg aac      912
   Gly Glu Thr Ala Thr Phe Gln Asp Trp Ile Gly Val Asn Tyr Val Asn
       290                 295                 300
   atc cgc cgc aca tcc aag gca tga                                      936
   Ile Arg Arg Thr Ser Lys Ala
   305                 310
<210> 10
   <211> 936
   <212> DNA
   <213> Artificial sequence
<220>
   <223> H305
<220>
   <221> exon
   <222> (1)..(933)
   <223>
<400> 10
   atg gac acg acg gtg ccc acc ttc agc ctg gcc gaa ctc cag cag ggc       48
   Met Asp Thr Thr Val Pro Thr Phe Ser Leu Ala Glu Leu Gln Gln Gly
   1               5                   10                  15
   ctg cac cag gac gag ttc cgc agg tgt ctg agg gac aag ggc ctc ttc       96
   Leu His Gln Asp Glu Phe Arg Arg Cys Leu Arg Asp Lys Gly Leu Phe
               20                  25                  30
   tat ctg acg gac tgc ggt ctg acc gac acc gag ctg aag tcg gcc aag      144
   Tyr Leu Thr Asp Cys Gly Leu Thr Asp Thr Glu Leu Lys Ser Ala Lys
           35                  40                  45
   gac atc gtc atc gac ttc ttc gag cac ggc agc gag gcg gag aag cgc      192
   Asp Ile Val Ile Asp Phe Phe Glu His Gly Ser Glu Ala Glu Lys Arg
       50                  55                  60
   gcc gtc acc tcg ccc gtc ccc acc acg cgc cgc ggc ttc acc ggg ctg      240
   Ala Val Thr Ser Pro Val Pro Thr Thr Arg Arg Gly Phe Thr Gly Leu
   65                  70                  75                  80
   gag tcg gag agc acc gcc cag atc acc aat acc ggc agc tac tcc gac      288
   Glu Ser Glu Ser Thr Ala Gln Ile Thr Asn Thr Gly Ser Tyr Ser Asp
                   85                  90                  95
   tac tcg atg tgc tac tcg atg ggc acc gcg gac aac ctc ttc ccg tcc      336
   Tyr Ser Met Cys Tyr Ser Met Gly Thr Ala Asp Asn Leu Phe Pro Ser
               100                 105                 110
   ggt gac ttc gag cgg atc tgg acc cag tac ttc gac cgc cag tac acc      384
   Gly Asp Phe Glu Arg Ile Trp Thr Gln Tyr Phe Asp Arg Gln Tyr Thr
           115                 120                 125
   gcc tcc cgc gcg gtc gcc cgg gag gtc ctg cgg gcg acc ggg acc gag      432
   Ala Ser Arg Ala Val Ala Arg Glu Val Leu Arg Ala Thr Gly Thr Glu
       130                 135                 140
   ccc gac ggc ggg gtc gag gcc ttc ctc gac tgc gag ccg ctg ctg cgg      480
   Pro Asp Gly Gly Val Glu Ala Phe Leu Asp Cys Glu Pro Leu Leu Arg
   145                 150                 155                 160
   ttc cgc tac ttc ccg cag gtc ccc gag cac cgc agc gcc gag gag cag      528
   Phe Arg Tyr Phe Pro Gln Val Pro Glu His Arg Ser Ala Glu Glu Gln
                   165                 170                 175
   ccc ctg cgg atg gcg ccg cac tac gac ctg tcg atg gtc acc ctc atc      576
   Pro Leu Arg Met Ala Pro His Tyr Asp Leu Ser Met Val Thr Leu Ile
               180                 185                 190
   cag cag aca ccc tgc gcc aac ggc ttc gtc agc ctc cag gcc gag gtc      624
   Gln Gln Thr Pro Cys Ala Asn Gly Phe Val Ser Leu Gln Ala Glu Val
           195                 200                 205
   ggc ggc gcg ttc acg gac ctg ccc tac cgt ccg gac gcc gtc ctc gtc      672
   Gly Gly Ala Phe Thr Asp Leu Pro Tyr Arg Pro Asp Ala Val Leu Val
       210                 215                 220
   ttc tgc ggc gcc atc gcg acc ctg gtg acc ggc ggc cag gtc aag gcc      720
   Phe Cys Gly Ala Ile Ala Thr Leu Val Thr Gly Gly Gln Val Lys Ala
   225                 230                 235                 240
   ccc cgg cac cat gtc gcg gcc ccc cgc agg gac cag ata gcg ggc agc      768
   Pro Arg His His Val Ala Ala Pro Arg Arg Asp Gln Ile Ala Gly Ser
                   245                 250                 255
   agc cgc acc tcc agt gtg ttc ttc ctc cgt ccc aac gcg gac ttc acc      816
   Ser Arg Thr Ser Ser Val Phe Phe Leu Arg Pro Asn Ala Asp Phe Thr
               260                 265                 270
   ttc tcc gtc ccg ctg gcg cgc gag tgc ggc ttc gat gtc agc ctg gac      864
   Phe Ser Val Pro Leu Ala Arg Glu Cys Gly Phe Asp Val Ser Leu Asp
           275                 280                 285
   ggc gag acc gcc acg ttc cag gat tgg atc ggg gtc aac tac gtg aac      912
   Gly Glu Thr Ala Thr Phe Gln Asp Trp Ile Gly Val Asn Tyr Val Asn
       290                 295                 300
   atc cgc cgc aca tcc aag gca tag                                      936
   Ile Arg Arg Thr Ser Lys Ala
   305                 310
<210> 11
   <211> 939
   <212> DNA
   <213> Artificial sequence
<220>
   <223> H307
<220>
   <221> exon
   <222> (1)..(936)
   <223>
<400> 11
   atg acg gac gcg acc gtg ccg acc ttc gat ctg gcc gag ctg cgt gag      48
   Met Thr Asp Ala Thr Val Pro Thr Phe Asp Leu Ala Glu Leu Arg Glu
   1               5                   10                  15
   ggc ttg cac cag gag gag ttc cgc cac tgc ctg cgc gag aag ggc gtg      96
   Gly Leu His Gln Glu Glu Phe Arg His Cys Leu Arg Glu Lys Gly Val
               20                  25                  30
   ttc tac ctc aag ggc acc ggg ctc gcc gag gcg gac cac gcc tcg gcg     144
   Phe Tyr Leu Lys Gly Thr Gly Leu Ala Glu Ala Asp His Ala Ser Ala
           35                  40                  45
   cgg gag atc gcg gtg gac ttc ttc gag cac ggc agc gag gcg gag aag     192
   Arg Glu Ile Ala Val Asp Phe Phe Glu His Gly Ser Glu Ala Glu Lys
       50                  55                  60
   cgc gcc gtc acc tcg ccc gtc ccc acc acg cgc cgc ggc ttc acc ggg     240
   Arg Ala Val Thr Ser Pro Val Pro Thr Thr Arg Arg Gly Phe Thr Gly
   65                  70                  75                  80
   ctg gag tcg gag agc acc gcg cag atc acg aac acc ggc agc tac tcc     288
   Leu Glu Ser Glu Ser Thr Ala Gln Ile Thr Asn Thr Gly Ser Tyr Ser
                   85                  90                  95
   gac tac tcg atg tgc tac tcg atg ggc acc gcg gac aac ctc ttc ccg     336
   Asp Tyr Ser Met Cys Tyr Ser Met Gly Thr Ala Asp Asn Leu Phe Pro
               100                 105                 110
   tcc ggt gac ttc gag cgg atc tgg acc cag tac ttc gac cgc cag tac     384
   Ser Gly Asp Phe Glu Arg Ile Trp Thr Gln Tyr Phe Asp Arg Gln Tyr
           115                 120                 125
   acc gcc tcc cgc gcg gtc gcc cgg gag gtc ctg cgg gcg acc ggg acc     432
   Thr Ala Ser Arg Ala Val Ala Arg Glu Val Leu Arg Ala Thr Gly Thr
       130                 135                 140
   gag ccc gac ggc ggg gtc gag gcc ttc ctc gac tgc gag ccg ctg ctg     480
   Glu Pro Asp Gly Gly Val Glu Ala Phe Leu Asp Cys Glu Pro Leu Leu
   145                 150                 155                 160
   cgg ttc cgc tac ttc ccg cag gtg ccc gag gat cgc gtg gcc gag gag     528
   Arg Phe Arg Tyr Phe Pro Gln Val Pro Glu Asp Arg Val Ala Glu Glu
                   165                 170                 175
   cag ccg ctg cgg atg gcc ccg cac tac gac ctc tcg atc gtc acc ctg      576
   Gln Pro Leu Arg Met Ala Pro His Tyr Asp Leu Ser Ile Val Thr Leu
               180                 185                 190
   atc cag cag aca ccc tgc gcc aac ggc ttc gtc agc ctc cag gcc gag      624
   Ile Gln Gln Thr Pro Cys Ala Asn Gly Phe Val Ser Leu Gln Ala Glu
           195                 200                 205
   gtc ggc ggc gcg ttc acg gac ctg ccc tac cgt ccg gac gcc gtc ctc      672
   Val Gly Gly Ala Phe Thr Asp Leu Pro Tyr Arg Pro Asp Ala Val Leu
       210                 215                 220
   gtc ttc tgc ggc gcc atc gcg acc ctg gtg acc ggc ggc cag gtc aag      720
   Val Phe Cys Gly Ala Ile Ala Thr Leu Val Thr Gly Gly Gln Val Lys
   225                 230                 235                 240
   gcc ccc cgg cac cat gtc gcg gcc ccc cgc agg gac cag ata gcg ggc      768
   Ala Pro Arg His His Val Ala Ala Pro Arg Arg Asp Gln Ile Ala Gly
                   245                 250                 255
   agc agc cgc acc tcc agc gtg ttc ttc ctg cgc ccc aac ggg gac ttc      816
   Ser Ser Arg Thr Ser Ser Val Phe Phe Leu Arg Pro Asn Gly Asp Phe
               260                 265                 270
   cgc ttc tcg gtg ccg cgg gcc agg gag tgc ggg ttc gac gtc ggc ctg      864
   Arg Phe Ser Val Pro Arg Ala Arg Glu Cys Gly Phe Asp Val Gly Leu
           275                 280                 285
   gac ggc gag acc gcc acg ttc cgg gat tgg atc ggg ggc aac tac gtg      912
   Asp Gly Glu Thr Ala Thr Phe Arg Asp Trp Ile Gly Gly Asn Tyr Val
       290                 295                 300
   aac atc cgc cgc aca tcc aag gca tag                                  939
   Asn Ile Arg Arg Thr Ser Lys Ala
   305                 310
<210> 12
   <211> 939
   <212> DNA
   <213> Artificial sequence
<220>
   <223> H308
<220>
   <221> exon
   <222> (1)..(936)
   <223>
<400> 12
   atg acg gac gcg acc gtg ccg acc ttc gat ctg gcc gag ctg cgt gag       48
   Met Thr Asp Ala Thr Val Pro Thr Phe Asp Leu Ala Glu Leu Arg Glu
   1               5                   10                  15
   ggc ttg cac cag gag gag ttc cgc cac tgc ctg cgc gag aag ggc gtg        96
   Gly Leu His Gln Glu Glu Phe Arg His Cys Leu Arg Glu Lys Gly Val
               20                  25                  30
   ttc tac ctc aag ggc acc ggg ctc gcc gag gcg gac cac gcc tcg gcg       144
   Phe Tyr Leu Lys Gly Thr Gly Leu Ala Glu Ala Asp His Ala Ser Ala
           35                  40                  45
   cgg gag atc gcg gtg gac ttc ttc gag cac ggc agc gag gcg gag aag       192
   Arg Glu Ile Ala Val Asp Phe Phe Glu His Gly Ser Glu Ala Glu Lys
       50                  55                  60
   cgc gcc gtc acc tcg ccc gtc ccc acc atg cgc cgc ggc ttc acc ggg       240
   Arg Ala Val Thr Ser Pro Val Pro Thr Met Arg Arg Gly Phe Thr Gly
   65                  70                  75                  80
   ctg gag tcg gag agc acc gcg cag atc acg aac acc ggc agc tac tcc       288
   Leu Glu Ser Glu Ser Thr Ala Gln Ile Thr Asn Thr Gly Ser Tyr Ser
                   85                  90                  95
   gac tac tcg atg tgc tac tcg atg ggc acc gcg gac aac ctc ttc ccg       336
   Asp Tyr Ser Met Cys Tyr Ser Met Gly Thr Ala Asp Asn Leu Phe Pro
               100                 105                 110
   tcc ggt gac ttc gag cgg atc tgg acc cag tac ttc gac cgc cag tac       384
   Ser Gly Asp Phe Glu Arg Ile Trp Thr Gln Tyr Phe Asp Arg Gln Tyr
           115                 120                 125
   acc gcc tcc cgc gcg gtc gcc cgg gag gtc ctg cgg gcg acc ggg acc       432
   Thr Ala Ser Arg Ala Val Ala Arg Glu Val Leu Arg Ala Thr Gly Thr
       130                 135                 140
   gag ccc gac ggc ggg gtc gag gcc ttc ctc gac tgc gag ccg ctg ctg       480
   Glu Pro Asp Gly Gly Val Glu Ala Phe Leu Asp Cys Glu Pro Leu Leu
   145                 150                 155                 160
   cgg ttc cgc tac ttc ccg cag gtg ccc gag gat cgc gtg gcc gag gag       528
   Arg Phe Arg Tyr Phe Pro Gln Val Pro Glu Asp Arg Val Ala Glu Glu
                   165                 170                 175
   cag ccg ctg cgg atg gcc ccg cac tac gac ctc tcg atc gtc acc ctg       576
   Gln Pro Leu Arg Met Ala Pro His Tyr Asp Leu Ser Ile Val Thr Leu
               180                 185                 190
   atc cag cag aca ccc tgc gcc aac ggc ttc gtc agc ctc cag gcc gag       624
   Ile Gln Gln Thr Pro Cys Ala Asn Gly Phe Val Ser Leu Gln Ala Glu
           195                 200                 205
   gtc ggc ggc gcg ttc acg gac ctg ccc tac cgt ccg gac gcc gtc ctc       672
   Val Gly Gly Ala Phe Thr Asp Leu Pro Tyr Arg Pro Asp Ala Val Leu
       210                 215                 220
   gtc ttc tgc ggc gcc atc gcg acc ctg gtg acc ggc ggc cag gtc aag       720
   Val Phe Cys Gly Ala Ile Ala Thr Leu Val Thr Gly Gly Gln Val Lys
   225                 230                 235                 240
   gcc ccc cgg cac cat gtc gcg gcc ccc cgc agg gac cag ata gcg ggc       768
   Ala Pro Arg His His Val Ala Ala Pro Arg Arg Asp Gln Ile Ala Gly
                   245                 250                 255
   agc agc cgc acc tcc agc gtg ttc ttc ctg cgc ccc aac ggg gac ttc       816
   Ser Ser Arg Thr Ser Ser Val Phe Phe Leu Arg Pro Asn Gly Asp Phe
               260                 265                 270
   cgc ttc tcg gtg ccg cgg gcc agg gag tgc ggg ttc aac gtc ggc ctg       864
   Arg Phe Ser Val Pro Arg Ala Arg Glu Cys Gly Phe Asn Val Gly Leu
           275                 280                 285
   gac ggc gag acc gcc acg ttc cgg gat tgg atc ggg ggc aac tac gtg       912
   Asp Gly Glu Thr Ala Thr Phe Arg Asp Trp Ile Gly Gly Asn Tyr Val
       290                 295                 300
   aac atc cgc cgc aca tcc aag gca tag                                   939
   Asn Ile Arg Arg Thr Ser Lys Ala
   305                 310
<210> 13
   <211> 939
   <212> DNA
   <213> Artificial sequence
<220>
   <223> H309
<220>
   <221> exon
   <222> (1)..(936)
   <223>
<400> 13
   atg acg gac gcg acc gtg ccg acc ttc gat ctg gcc gag ctg cgt gag        48
   Met Thr Asp Ala Thr Val Pro Thr Phe Asp Leu Ala Glu Leu Arg Glu
   1               5                   10                  15
   ggc ttg cac cag gag gag ttc cgc cac tgc ctg cgc gag aag ggc gtg        96
   Gly Leu His Gln Glu Glu Phe Arg His Cys Leu Arg Glu Lys Gly Val
               20                  25                  30
   ttc tac ctc aag ggc acc ggg ctc gcc gag gcg gac cac gcc tcg gcg       144
   Phe Tyr Leu Lys Gly Thr Gly Leu Ala Glu Ala Asp His Ala Ser Ala
           35                  40                  45
   cgg gag atc gcg gtg gac ttc ttc gag cac ggc agc gag gcg gag aag       192
   Arg Glu Ile Ala Val Asp Phe Phe Glu His Gly Ser Glu Ala Glu Lys
       50                  55                  60
   cgc gcc gtc acc tcg ccc gtc ccc acc acg cgc cgc ggc ttc acc ggg       240
   Arg Ala Val Thr Ser Pro Val Pro Thr Thr Arg Arg Gly Phe Thr Gly
   65                  70                  75                  80
   ctg gag tcg gag agc acc gcg cag atc acg aac acc ggc agc tac tcc       288
   Leu Glu Ser Glu Ser Thr Ala Gln Ile Thr Asn Thr Gly Ser Tyr Ser
                   85                  90                  95
   gac tac tcg atg tgc tac tcg atg ggc acc gcg gac aac ctc ttc ccg        336
   Asp Tyr Ser Met Cys Tyr Ser Met Gly Thr Ala Asp Asn Leu Phe Pro
               100                 105                 110
   tcc ggt gac ttc gag cgg atc tgg acc cag tac ttc gac cgc cag tac        384
   Ser Gly Asp Phe Glu Arg Ile Trp Thr Gln Tyr Phe Asp Arg Gln Tyr
           115                 120                 125
   acc gcc tcc cgc gcg gtc gcc cgg gag gtc ctg cgg gcg acc ggg acc        432
   Thr Ala Ser Arg Ala Val Ala Arg Glu Val Leu Arg Ala Thr Gly Thr
       130                 135                 140
   gag ccc gac ggc ggg gtc gag gcc ttc ctc gac tgc gag ccg ctg ctg        480
   Glu Pro Asp Gly Gly Val Glu Ala Phe Leu Asp Cys Glu Pro Leu Leu
   145                 150                 155                 160
   cgg ttc cgc tac ttc ccg cag gtg ccc gag gat cgc gtg gcc gag gag        528
   Arg Phe Arg Tyr Phe Pro Gln Val Pro Glu Asp Arg Val Ala Glu Glu
                   165                 170                 175
   cag ccg ctg cgg atg gcc ccg cac tac gac ctc tcg atc gtc acc ctg        576
   Gln Pro Leu Arg Met Ala Pro His Tyr Asp Leu Ser Ile Val Thr Leu
               180                 185                 190
   atc cag cag aca ccc tgc gcc aac ggc ttc gtc agc ctc cag gcc gag        624
   Ile Gln Gln Thr Pro Cys Ala Asn Gly Phe Val Ser Leu Gln Ala Glu
           195                 200                 205
   gtc ggc ggc gcg ttc acg gac ctg ccc tac cgt ccg gac gcc gtc ctc        672
   Val Gly Gly Ala Phe Thr Asp Leu Pro Tyr Arg Pro Asp Ala Val Leu
       210                 215                 220
   gtc ttc tgc ggc gcc atc gcg acc ctg gtg acc ggc ggc cag gtc aag        720
   Val Phe Cys Gly Ala Ile Ala Thr Leu Val Thr Gly Gly Gln Val Lys
   225                 230                 235                 240
   gcc ccc cgg cac cat gtc gcg gcc ccc cgc agg gac cag ata gcg ggc        768
   Ala Pro Arg His His Val Ala Ala Pro Arg Arg Asp Gln Ile Ala Gly
                   245                 250                 255
   agc agc cgc acc tcc agc gtg ttc ttc ctg cgc ccc aac ggg gac ttc        816
   Ser Ser Arg Thr Ser Ser Val Phe Phe Leu Arg Pro Asn Gly Asp Phe
               260                 265                 270
   cgc ttc tcg gtg ccg cgg gcc agg gag tgc ggg ttc aac gtc ggc ctg        864
   Arg Phe Ser Val Pro Arg Ala Arg Glu Cys Gly Phe Asn Val Gly Leu
           275                 280                 285
   gac ggc gag acc gcc acg ttc cgg gat tgg atc ggg ggc aac tac gtg        912
   Asp Gly Glu Thr Ala Thr Phe Arg Asp Trp Ile Gly Gly Asn Tyr Val
       290                 295                 300
   aac atc cgc cgc aca tcc aag gca tag                                    939
   Asn Ile Arg Arg Thr Ser Lys Ala
   305                 310
<210> 14
   <211> 5335
   <212> DNA
   <213> Artificial sequence
<220>
   <223> Penicillium expression cassette plasmid
<220>
   <221> exon
   <222> (930)..(1862)
   <223> Coding sequence expandase
<400> 14
   gaattcctta tactgggcct gctgcattgg tctgccattg cagggtatat atggctgacc      60
   tggccaatct ccatcgagaa tctgggcgac tgaagaactg cccgcagaca agatggagac     120
   tttcgtctag cacggtctag ggcagatccg atgccattgg ctctgtcaac tgtcgactac     180
   atgtatctgc atgttgcatc gggaaatccc accacaggga cagccaagcg gccccgcgac     240
   ttggcagtgg gcaaactacg cccgattctg gtgccaagaa ccgagaagaa tgagacagac     300
   ccacgttgca ctctaaccgg atgctatcga cttacggtgg ctgaagattc aacacgctgc     360
   aacgagagcc aaggtggtcc ggacattttc tacgtgccgg tttaccttgg aacatcgccg     420
   tcgttgagtg cacgttgcct actctctcgt ggcttggctg ggcccacgag cccgattgac     480
   tcgacggtgt tacttgggta tctatggccc cgttttctgg cacggtaatg ataagtactt     540
   actagtcttc gagcggggga gtgttgctct gcccgagcat caacgattgg cctgatcgca     600
   ccgtctgcaa atgccacggt gcggaccgac tgaaatctca gaccaccaaa gaccctccga     660
   cttcgagata cggttactaa ttttacactg gctccagcgg ccccatccag taagcatctg     720
   ggctgcaagc gtataatgtc tccaggttgt ctcagcataa acaccccgcc cccgctcagg     780
   cacacaggaa gagagctcag gtcgtttcca ttgcgtccat actcttcact cattgtcatc     840
   tgcaggagaa cttcccctgt ccctttgcca agccctctct tcgtcgttgt ccacgccttc     900
   aagttttcac cattattttt ctagaccat atg gac acg acg gtg ccc acc ttc       953
                                   Met Asp Thr Thr Val Pro Thr Phe
                                   1               5
   agc ctg gcc gaa ctc cag cag ggc ctg cgc cag gac gag ttc cgc agg      1001
   Ser Leu Ala Glu Leu Gln Gln Gly Leu Arg Gln Asp Glu Phe Arg Arg
       10                  15                  20
   tgt ctg agg gac aag ggc ctc ttc tat ctg acg gac tgc ggt ctg acc      1049
   Cys Leu Arg Asp Lys Gly Leu Phe Tyr Leu Thr Asp Cys Gly Leu Thr
   25                  30                  35                  40
   gac acc gag ctg aag tcg gcc aag gac atc gtc atc gac ttc ttc gag      1097
   Asp Thr Glu Leu Lys Ser Ala Lys Asp Ile Val Ile Asp Phe Phe Glu
                   45                  50                  55
   cac ggc agc gag gcg gag aag cgc gcc gtc acc tcg ccc gtc ccc acc      1145
   His Gly Ser Glu Ala Glu Lys Arg Ala Val Thr Ser Pro Val Pro Thr
               60                  65                  70
   atg cgc cgc ggc ttc acc ggg ctg gag tcg gag agc acc gcc cag att      1193
   Met Arg Arg Gly Phe Thr Gly Leu Glu Ser Glu Ser Thr Ala Gln Ile
           75                  80                  85
   acc aat acc ggc agc tac tcc gac tac tcg atg tgc tac tcg atg ggc      1241
   Thr Asn Thr Gly Ser Tyr Ser Asp Tyr Ser Met Cys Tyr Ser Met Gly
       90                  95                  100
   acc gcg gac aac ctc ttc ccg tcc ggt gac ttc gag cgg atc tgg acc      1289
   Thr Ala Asp Asn Leu Phe Pro Ser Gly Asp Phe Glu Arg Ile Trp Thr
   105                 110                 115                 120
   cag tac ttc gac cgc cag tac acc gcc tcc cgc gcg gtc gcc cgg gag      1337
   Gln Tyr Phe Asp Arg Gln Tyr Thr Ala Ser Arg Ala Val Ala Arg Glu
                   125                 130                 135
   gtc ctg cgg gcg acc ggg acc gag ccc gac ggc ggg gtc gag gcc ttc      1385
   Val Leu Arg Ala Thr Gly Thr Glu Pro Asp Gly Gly Val Glu Ala Phe
               140                 145                 150
   ctc gac tgc gag ccg ctg ctg cgg ttc cgc tac ttc ccg cag gtc ccc      1433
   Leu Asp Cys Glu Pro Leu Leu Arg Phe Arg Tyr Phe Pro Gln Val Pro
           155                 160                 165
   gag cac cgc agc gcc gag gag cag ccc ctg cgg atg gcg ccg cac tac      1481
   Glu His Arg Ser Ala Glu Glu Gln Pro Leu Arg Met Ala Pro His Tyr
       170                 175                 180
   gac ctg tcg atg gtc acc ctc atc cag cag aca ccc tgc gcc aac ggc      1529
   Asp Leu Ser Met Val Thr Leu Ile Gln Gln Thr Pro Cys Ala Asn Gly
   185                 190                 195                 200
   ttc gtc agc ctc cag gcc gag gtc ggc ggc gcg ttc acg gac ctg ccc      1577
   Phe Val Ser Leu Gln Ala Glu Val Gly Gly Ala Phe Thr Asp Leu Pro
                   205                 210                 215
   tac cgt ccg gac gcc gtc ctc gtc ttc tgc ggc gcc atc gcg acc ctg      1625
   Tyr Arg Pro Asp Ala Val Leu Val Phe Cys Gly Ala Ile Ala Thr Leu
               220                 225                 230
   gtg acc ggc ggc cag gtc aag gcc ccc cgg cac cat gtc gcg gcc ccc      1673
   Val Thr Gly Gly Gln Val Lys Ala Pro Arg His His Val Ala Ala Pro
           235                 240                 245
   cgc agg gac cag ata gcg ggc agc agc cgc acc tcc agt gtg ttc ttc      1721
   Arg Arg Asp Gln Ile Ala Gly Ser Ser Arg Thr Ser Ser Val Phe Phe
       250                 255                 260
   ctc cgt ccc aac gcg gac ttc acc ttc tcc gtc ccg ctg gcg cgc gag      1769
   Leu Arg Pro Asn Ala Asp Phe Thr Phe Ser Val Pro Leu Ala Arg Glu
   265                 270                 275                 280
   tgc ggc ttc aat gtc agc ctg gac ggc gag acc gcc acg ttc cag gat      1817
   Cys Gly Phe Asn Val Ser Leu Asp Gly Glu Thr Ala Thr Phe Gln Asp
                   285                 290                 295
   tgg atc ggg gtc aac tac gtg aac atc cgc cgc aca tcc aag gca          1862
   Trp Ile Gly Val Asn Tyr Val Asn Ile Arg Arg Thr Ser Lys Ala
               300                 305                 310
   tgataggctc ttcatgacga gaatatgcat cttttgtatg tagcttcaac cgactccgtc    1922
   ttcacttctt cgcccgcact gcctaccgtt tgtaccatct gactcatata aatgtctagc    1982
   ccctacctac actataccta agggagagaa gcgtagagtg attaacgtac gggcctatag    2042
   taccccgatc tctagataga acatttagta gagattagga tgcctaacta atttaacttg    2102
   agcattgtcc cgttcatatt gattttcagt ccattataca ctcttaatcg tttcccggta    2162
   gaagcctgat atatacgacc atagggtgtg gagaacaggg cttcccgtct gcttggccgt    2222
   acttaagcta tatattctac acggccaata ctcaatgtgc ccttagcacc taagcggcac    2282
   tctagggtaa gtgcgggtga tataggtgag aagtcttaag actgaagaca ggatatcacg    2342
   cgttaccctg caccgtacct actaccttca atatcaactc tttcaggatg gacagggtcg    2402
   acactagttc tagagcggcc gccaccgcgg tggagctcca gcttttgttc cctttagtga    2462
   gggttaattg cgcgcttggc gtaatcatgg tcatagctgt ttcctgtgtg aaattgttat    2522
   ccgctcacaa ttccacacaa catacgagcc ggaagcataa agtgtaaagc ctggggtgcc    2582
   taatgagtga gctaactcac attaattgcg ttgcgctcac tgcccgcttt ccagtcggga    2642
   aacctgtcgt gccagctgca ttaatgaatc ggccaacgcg cggggagagg cggtttgcgt    2702
   attgggcgct cttccgcttc ctcgctcact gactcgctcg gctcggtcgt tcggctgcgg    2762
   cgagcggtat cagctcactc aaaggcggta atacggttat ccacagaatc aggggataac    2822
   gcaggaaaga acatgtgagc aaaaggccag caaaaggcca ggaaccgtaa aaaggccgcg    2882
   ttgctggcgt ttttccatag gctccgcccc cctgacgagc atcacaaaaa tcgacgctca    2942
   agtcagaggt ggcgaaaccc gacaggacta taaagatacc aggcgtttcc ccctggaagc    3002
   tccctcgtgc gctctcctgt tccgaccctg ccgcttaccg gatacctgtc cgcctttctc    3062
   ccttcgggaa gcgtggcgct ttctcatagc tcacgctgta ggtatctcag ttcggtgtag    3122
   gtcgttcgct ccaagctggg ctgtgtgcac gaaccccccg ttcagcccga ccgctgcgcc    3182
   ttatccggta actatcgtct tgagtccaac ccggtaagac acgacttatc gccactggca 3242
   gcagccactg gtaacaggat tagcagagcg aggtatgtag gcggtgctac agagttcttg 3302
   aagtggtggc ctaactacgg ctacactaga aggacagtat ttggtatctg cgctctgctg 3362
   aagccagtta ccttcggaaa aagagttggt agctcttgat ccggcaaaca aaccaccgct 3422
   ggtagcggtg gtttttttgt ttgcaagcag cagattacgc gcagaaaaaa aggatctcaa 3482
   gaagatcctt tgatcttttc tacggggtct gacgctcagt ggaacgaaaa ctcacgttaa 3542
   gggattttgg tcatgagatt atcaaaaagg atcttcacct agatcctttt aaattaaaaa 3602
   tgaagtttta aatcaatcta aagtatatat gagtaaactt ggtctgacag ttaccaatgc 3662
   ttaatcagtg aggcacctat ctcagcgatc tgtctatttc gttcatccat agttgcctga 3722
   ctccccgtcg tgtagataac tacgatacgg gagggcttac catctggccc cagtgctgca 3782
   atgataccgc gagacccacg ctcaccggct ccagatttat cagcaataaa ccagccagcc 3842
   ggaagggccg agcgcagaag tggtcctgca actttatccg cctccatcca gtctattaat 3902
   tgttgccggg aagctagagt aagtagttcg ccagttaata gtttgcgcaa cgttgttgcc 3962
   attgctacag gcatcgtggt gtcacgctcg tcgtttggta tggcttcatt cagctccggt 4022
   tcccaacgat caaggcgagt tacatgatcc cccatgttgt gcaaaaaagc ggttagctcc 4082
   ttcggctctc cgatcgttgt cagaagtaag ttggccgcag tgttatcact catggttatg 4142
   gcagcactgc ataattctct tactgtcatg ccatccgtaa gatgcttttc tgtgactggt 4202
   gagtactcaa ccaagtcatt ctgagaatag tgtatgcggc gaccgagttg ctcttgcccg 4262
   gcgtcaatac gggataatac cgcgccacat agcagaactt taaaagtgct catcattgga 4322
   aaacgttctt cggggcgaaa actctcaagg atcttaccgc tgttgagatc cagttcgatg 4382
   taacccactc gtgcacccaa ctgatcttca gcatctttta ctttcaccag cgtttctggg 4442
   tgagcaaaaa caggaaggca aaatgccgca aaaaagggaa taagggcgac acggaaatgt 4502
   tgaatactca tactcttcct ttttcaatat tattgaagca tttatcaggg ttattgtctc 4562
   atgagcggat acatatttga atgtatttag aaaaataaac aaataggggt tccgcgcaca 4622
   tttccccgaa aagtgccacc tgacgcgccc tgtagcggcg cattaagcgc ggcgggtgtg 4682
   gtggttacgc gcagcgtgac cgctacactt gccagcgccc tagcgcccgc tcctttcgct 4742
   ttcttccctt cctttctcgc cacgttcgcc ggctttcccc gtcaagctct aaatcggggg 4802
   ctccctttag ggttccgatt tagtgcttta cggcacctcg accccaaaaa acttgattag 4862
   ggtgatggtt cacgtagtgg gccatcgccc tgatagacgg tttttcgccc tttgacgttg 4922
   gagtccacgt tctttaatag tggactcttg ttccaaactg gaacaacact caaccctatc   4982
   tcggtctatt cttttgattt ataagggatt ttgccgattt cggcctattg gttaaaaaat   5042
   gagctgattt aacaaaaatt taacgcgaat tttaacaaaa tattaacgct tacaatttcc   5102
   attcgccatt caggctgcgc aactgttggg aagggcgatc ggtgcgggcc tcttcgctat   5162
   tacgccagct ggcgaaaggg ggatgtgctg caaggcgatt aagttgggta acgccagggt   5222
   tttcccagtc acgacgttgt aaaacgacgg ccagtgagcg cgcgtaatac gactcactat   5282
   agggcgaatt gggtaccggg ccccccctcg agtgcggccg caagcttgat atc          5335

## Description

### Field of the invention

The present invention relates to mutant expandase enzymes, polynucleotides encoding such enzymes, to microorganisms transformed with said polynucleotides encoding said mutant expandase enzymes and with the use of such mutant expandase enzymes or such microorganisms in the ring expansion of 5-carboxypentanoyl-6-aminopenicillanic acid (adipyl-6-APA = ad-6-APA).

### Background of the invention

Beta-lactam antibiotics constitute the most important group of antibiotic compounds with a long history of clinical use. Among this group, the prominent ones are the penicillins and cephalosporins. Penicillins are naturally produced by various filamentous fungi such as *Penicillium* (e.g. *P. chrysogenum).* Cephalosporins are naturally produced by various microorganisms such as *Acremonium* (e.g. *A*. *chrysogenum)* and *Streptomyces* (e.g. *Streptomyces clavuligerus)*

As a result of classical strain improvement techniques, the production levels of the antibiotics in *P. chrysogenum* and *A. chrysogenum* have increased remarkably over the past decades. With the increasing knowledge of the biosynthetic pathways leading to penicillins and cephalosporins, and the advent of recombinant DNA technology, new tools for the improvement of production strains have become available.

Most enzymes involved in β-lactam biosynthesis have been identified and their corresponding genes have been cloned, as can be found in Ingolia and Queener, Med Res Rev (1989) 9:245-264 (biosynthesis route and enzymes), and Aharonowitz, Cohen, and Martin, Ann Rev Microbiol (1992) 46:461-495 (gene cloning).

The first two steps in the biosynthesis of penicillin in *P. chrysogenum* are the condensation of the three amino acids L-5-amino-5-carboxypentanoic acid (L-α-aminoadipic acid) (A), L-cystein (C) and L-valine (V) into the tripeptide LLD-ACV, followed by cyclization of this tripeptide to form isopenicillin N. This compound contains the typical β-lactam structure.

The third step involves the replacement of the hydrophilic side chain of L-5-amino-5-carboxypentanoic acid by a hydrophobic side chain by the action of the enzyme acyltransferase (AT).

In EP-A-0448180 has been described that the enzymatic exchange reaction mediated by AT takes place inside a cellular organelle, the microbody. The observation that substantial quantities of deacetoxycephalosporin C (DAOC) can be formed by non-precursed *P. chrysogenum* transformants expressing deacetoxycephalosporin C synthase (EC 1.14.20.1 - DAOCS, further indicated herein as expandase) implies the presence of significant amounts of penicillin N, the natural substrate for expandase, in *P. chrysogenum* (Alvi et al., J Antibiot (1995) 48:338-340). However, the D-α-amino-adipyl side chains of DAOC cannot be easily removed.

Cephalosporins are much more expensive than penicillins. One reason is that some cephalosporins (e.g., cephalexin) are made from penicillins by a number of chemical conversions. Another reason is that, so far, only cephalosporins with a D-α-amino-adipyl side chain could be fermented. Cephalosporin C, by far the most important starting material in this respect, is very soluble in water at any pH, thus implying lengthy and costly isolation processes using cumbersome and expensive column technology. Cephalosporin C obtained in this way has to be converted into therapeutically used cephalosporins by a number of chemical and enzymatic conversions.

The methods currently favored in industry to prepare the intermediate 7-amino-deacetoxycephaloporanic acid (7-ADCA) involve complex chemical steps leading to the expansion and derivatization of penicillin G. One of the necessary chemical steps to produce 7-ADCA involves the expansion of the 5-membered penicillin ring structure to a 6-membered cephalosporin ring structure (see for instance US 4,003,894). This complex chemical processing is both expensive and noxious to the environment.

Consequently, there is a great desire to replace such chemical processes with enzymatic reactions such as enzymatic catalysis, preferably during fermentation. A key to the replacement of the chemical expansion process by a biological process is the central enzyme in the cephalosporin biosynthetic pathway, expandase.

The expandase enzyme from the bacterium *Streptomyces clavuligerus* (*S. clavuligerus*) was found to carry out, in some cases, penicillin ring expansions. When introduced into *P. chrysogenum,* it can convert the penicillin ring structure into the cephalosporin ring structure, as described in Cantwell et al., Proc R Soc Lond B (1992) 248:283-289. The expandase enzyme has been well characterized (EP-A-0366354) both biochemical and functional, as has its corresponding gene. Both physical maps of the *cefE* gene (the gene encoding the expandase enzyme of *S*. *clavuligerus -* EP-A-0341892), DNA sequence and transformation studies in *P. chrysogenum* with *cefE* have been described. The DNA and amino acid sequence of the *S*. *clavuligerus* expandase enzyme are represented in SEQ ID NO 1.

Another source for an expandase enzyme is the bacterium *Nocardia lactamdurans (N. lactamdurans,* formerly *S*. *lactamdurans).* Both the biochemical properties of the enzyme and the DNA sequence of the gene encoding the enzyme have been described - see Cortes et al., J Gen Microbiol (1987) 133:3165-3174 and Coque et al., Mol Gen Genet (1993) 236:453-458, respectively. The DNA and amino acid sequence of the *N. lactamdurans* expandase enzyme are represented in SEQ ID NO 2.

Recently, novel expandase (cefE) genes and enzymes have been found in *Streptomyces jumonjinensis, Streptomyces ambofaciens and Streptomyces chartreuses -* see Hsu et al. (2004), Appl. and Environm. Microbiol. 70, 6257-6263. Table 1 summarizes the amino acid sequence identities from several expandases and shows that the sequence identities range from 67 to 85%, depending on the source of the enzyme.

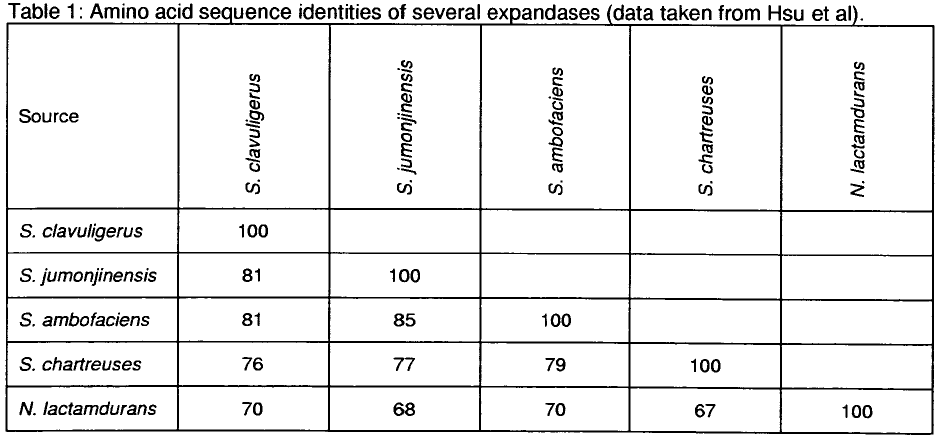

In the biosynthesis of cephalosporins, which takes mainly place in prokaryotic cells, the deacetoxycephalosporin-C is subsequently converted to deacetylcephalosporin-C by the enzyme deacetylcephalosporin C synthase also named deacetoxycephalosporin-C hydroxylase or hydroxylase (EC 1.14.11.26 - DACS). Genes encoding such hydroxylases are named *cefF*-genes (e.g. see Hsu et al.) The expandase found in eukaryotic cells, e.g. *Acremonium chrysogenum* can catalyze the direct conversion of penicillin N to deacetoxycephalosporin-C due to possession of both expandase and hydrolyase activity. hence the encoded gene is termed *cefEF* (see Hsu et al.).

As defined herein, the term expandase relates to expandase enzymes (EC 1.14.20.1, encoded by *cefE* genes) as well as expandases also possessing in addition the hydroxylase activity (EC 1.14.20.1 + EC 1.14.11.26 encoded by *cefEF* genes)

Since the expandase enzyme catalyses the expansion of the 5-membered thiazolidine ring of penicillin N to the 6-membered dihydrothiazine ring of DAOC, this enzyme would be of course a logical candidate to replace the ring expansion steps of the chemical process. Unfortunately, the enzyme works on the penicillin N intermediate of the cephalosporin biosynthetic pathway, but not or very inefficiently on the readily available inexpensive penicillins as produced by *P. chrysogenum,* like penicillin V or penicillin G. Penicillin N is commercially not available and even when expanded, its D-α-amino-adipyl side chain cannot be easily removed by penicillin acylases.

It has been reported that the expandase enzyme is capable of expanding penicillins with particular side chains to the corresponding 7-ADCA derivative. This feature of the expandase has been exploited in the technology as disclosed in EP-A-0532341, WO95/04148 and WO95/04149. In these disclosures the conventional chemical *in vitro* conversion of penicillin G to 7-ADCA has been replaced by the *in vivo* conversion of certain 6-aminopenicillanic acid (6-APA) derivatives in recombinant *Penicillium chrysogenum* strains transformed with an expandase gene.

More particularly, EP-A-0532341 teaches the *in vivo* use of the expandase enzyme in *P. chrysogenum,* in combination with a adipyl side chain (further referred to as adipyl) as a feedstock, which is a substrate for the acyltransferase enzyme in *P*. *chrysogenum.* This leads to the formation of adipyl-6-APA, which is converted by an expandase enzyme introduced into the *P. chrysogenum* strain to yield adipyl-7-ADCA. Finally, the removal of the adipyl side chain is described, yielding 7-ADCA as a final product. Furthermore, EP-A-540210 teaches the similar production of adipyl-7-ADAC and adipyl-7-ACA.

As an alternative approach, expansion of penicillin G using *P. chrysogenum* transformed with *cefE* has been proposed in EP0828850. Furthermore, in order to increase the expansion of penicillin G the use of mutant *cefE* gene of *S*. *clavuligerus* has been described. US 5,919,680, WO 98/02551, WO 99/33994, WO 01/85951 and EP 1348759 all disclose mutant *cefE* genes allegedly coding for enzymes having a higher expandase activity on penicillin G.

Nevertheless, these mutant expandases still do not provide for a commercially attractive process for the production of cephalosporins.

Therefore, there is a need for further improving the process wherein cephalosporins are prepared from expansion of adipyl-6-APA. This process is characterized by an efficient removal of the side chain material from the cephalosporin core material. One step to further improve this process is improvement of the expandase activity on adipyl-6-APA.

### Description of the Figures

1/3. A & B Amino acid alignment of improved expandase mutants; SCLAVEIW represents amino acid sequence of expandase of *S*. *clavuligerus;* NOCAEIW represents amino acid sequence of expandase of *N. lactamdurans;* For the other sequences the notation is as follows: 309EIWIT represents amino acid sequence of expandase of mutant expandase H309, and so son.
2/3. Schematic representation of vector pIATWAn
3/3. HPLC analysis of expandase assay mixture according to Example 3
   A. at t = 0 minutes
   B. at t = 30 minutes

### Detailed description of the invention

In a first aspect, the invention provides a mutant expandase that is a variant of a model polypeptide with expandase activity having the sequence as depicted in SEQ ID NO. 1 or an amino acid sequence having at least 70% identity with SEQ ID NO. 1, and whereby threonine at amino acid position 89 in the model peptide has been replaced by lysine using the amino acid position numbering of the amino acid sequence of the expandase enzyme as depicted in SEQ ID NO. 1. Preferably, the invention provides a mutant expandase, whereby the mutant expandase has an at least 2-fold improved *in vitro* expandase activity towards adipyl-6-APA in comparison with the model polypeptide with expandase activity of SEQ ID NO.1. The determination of the *in vitro* expandase activity towards adipyl-6-APA is described in detail in the Materials and Methods (Assay (1) - (3)). More preferably the *in vitro* expandase activity towards adipyl-6-APA of the mutant expandase is improved at least 2.5-fold, more preferably at least 3-fold, more preferably at least 4-fold, more preferably at least 5-fold, more preferably at least 6-fold, more preferably at least 7-fold, more preferably at least 8-fold, more preferably at least 9-fold, more preferably at least 10-fold, more preferably at least 11-fold.

In some of the mutant expandase enzymes described herein, parts of the amino acid sequence of the expandase of *S*. *clavuligerus* have been replaced by corresponding parts of the amino acid sequence of expandase of *N. lactamdurans.* The amino acid sequence of the *Cef*E gene of *N. lactamdurans* strain ATCC27382 is represented in SEQ ID NO: 2.

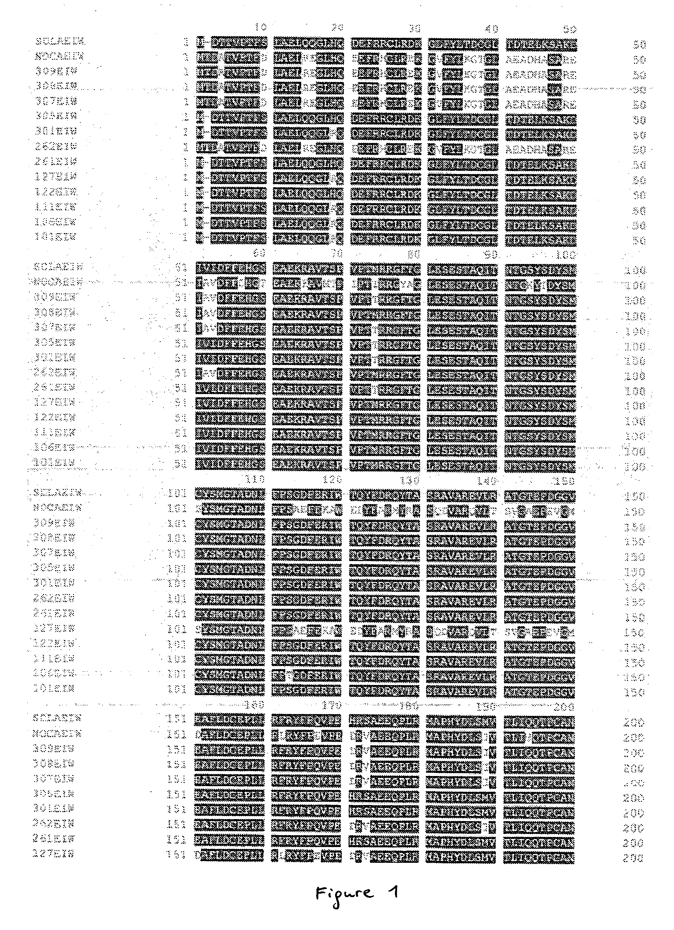

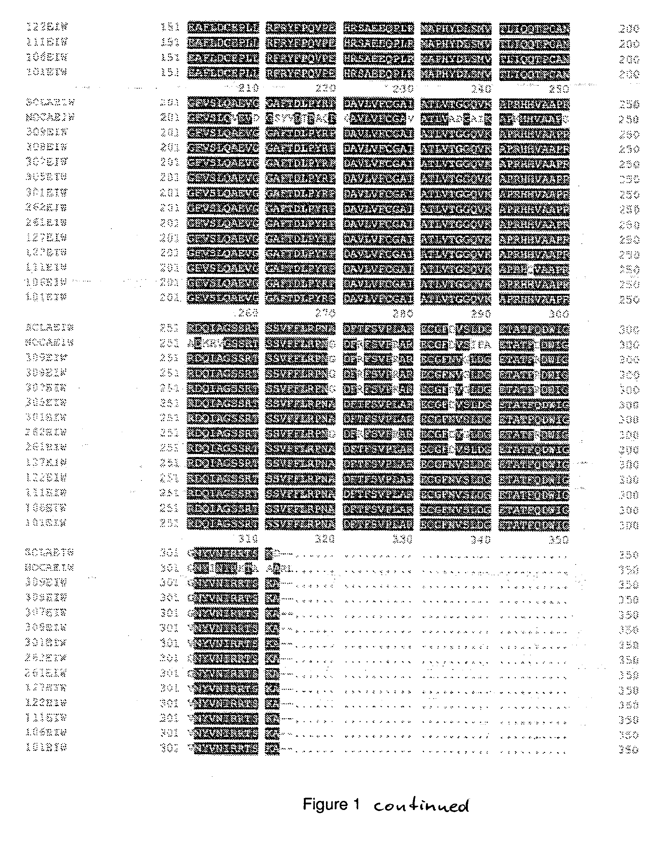

In the description of these mutant expandases the amino acids are numbered according to the numbering of the homologous parts of the amino acid sequence of the expandase of *S*. *clavuligerus.* The homology of the expandase amino acid sequences of S. *clavuligerus* and *N. lactamdurans* is to be derived from Figure 1 and Table 1.

With "altered or mutant expandase" in the context of the present invention is meant any enzyme having expandase activity, which has not been obtained from a natural source and for which the amino acid sequence differs from the complete amino acid sequences of the natural expandase enzymes of *S*. *clavuligerus* and *N. lactamdurans.*

The invention also provides a mutant expandase which is additionally being modified at least at an amino acid position selected from group 1 consisting of positions 2, 18, 59, 73, 74, 89, 90, 99, 101, 105, 112, 113, 155, 170, 177, 209, 213, 217, 244, 249, 251, 277, 278, 280, 281, 284, 293, 300, 307 and 311 using the amino acid position numbering of the amino acid sequence of the expandase enzyme encoded by the cefE gene of *Streptomyces clavuligerus.* The nucleotide sequence of the cefE gene of *Streptomyces clavuligerus* as well as the amino acid sequence encoded by said cefE gene are depicted in SEQ ID NO: 1. More preferably, the mutant expandase is modified at least at an amino acid position selected from group 2 consisting of positions 2, 18, 59, 89, 90, 99, 101, 105, 112, 113, 170, 177, 209, 213, 217, 249, 251, 278, 280, 284 and 293. The mutant expandase may also have been modified at least at an amino acid position selected from the group 1 which does not form part of group 2, together with at least an amino acid position selected from the group 2.

The modification at an amino acid position may comprise a substitution by another amino acid, selected from the group of 20 L-amino acids that occur in Nature - see Table 2. Alternatively, the modification at an amino acid position may comprise a deletion of the amino acid at said position. Furthermore, the modification at an amino acid position may comprise a substitution of one or more amino acids at the C-terminal or N-terminal side of said amino acid.

**Table 2**

| Amino Acid | 3-letter code | 1-letter code |
|---|---|---|
| Alanine | Ala | A |
| Arginine | Arg | R |
| Asparagine | Asn | N |
| Aspartic acid | Asp | D |
| Cystein | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophane | Trp | W |
| Tyrosine | Tyr | Y |
| Valine | Val | V |

The model polypeptide with expandase activity as used in the present invention is selected from the group consisting of a polypeptide with expandase activity, preferably having an amino acid sequence according to SEQ ID NO: 1 and polypeptides with expandase activity having an amino acid sequence with a percentage identity with SEQ ID NO: 1 of at least 70%, preferably at least 75%, more preferably at least 80%, more preferably at least 85%, more preferably at least 90%, most preferably at least 95%, such as the expandase enzymes that are summarized in Table 1. Most preferred as model polypeptide with expandase activity as used in the present invention is a polypeptide with expandase activity, having the amino acid sequence according to SEQ ID NO: 1 or having the amino acid sequence according to SEQ ID NO: 2.

The mutant expandases described herein have modifications at least at
○ 2 or more amino acid positions selected from the group consisting of 2, 89, 277, 281 and 300, more preferably at positions 2+281 or 89+281 or 277+300.
○ 3 or more amino acid positions selected from the group consisting of 2, 89, 281, 293 and 311, more preferably at positions 2+89+281 or 89+281+311 or 89+281 +293.
○ 4 or more amino acid positions selected from the group consisting of 2, 73, 89, 90, 217, 244, 277, 280, 281, 306, 307 and 311, more preferably at positions 2+277+280+281 or 2+281+307+311 or 73+89+281+311 or 89+217+281+311 or 89+244+281 +311 or 89+281+307+311 or 277+281+306+311 or 89+281+306+311 or 90+281+306+311.
○ 5 or more amino acid positions selected from the group consisting of 2, 73, 89, 90, 155, 213, 249, 278, 281, 293, 300, 306, 307 and 311, more preferably at . positions 2+89+281+306+311 or 2+155+281+306+311 or 73+89+213+281+311 or 89+78+218+307+311, 89+281 +293+300+311 or 89+249+281 +307+311.
○ 6 amino acid positions selected from the group consisting of 90+105+113+281+306+311.
○ 7 or more amino acid positions selected from the group consisting of 2, 73, 89, 105, 113, 155, 170, 177, 251, 277, 280, 281, 293, 300, 306, 307 and 311, more preferably 73+89+281 +293+300+307+311 or 105+113+155+177+281+306+311 or 155+177+277+280+281+306+ 311.
○ 8 or more amino acid positions selected from the group consisting of 2, 89, 90, 99, 105, 113, 155, 177, 277, 281, 307 and 311 more preferably at 2+90+99+105+113+281+306+311 or 2+90+105+113+155+177+277+281
○ 9 amino acid positions selected from the group consisting of 2+90+105+113+155+177+281+306+311.
○ 10 amino acid positions selected from the group consisting of 2, 59, 90, 101, 105, 113, 155, 177, 209, 277, 281, 307 and 311 more preferably at 2+90+105+113+155+177+277+281+306+311.
○ 11 amino acid positions selected from the group consisting of 2, 90, 105, 113, 155, 177, 277, 281, 293, 307, 311.

Substitutions of certain of the amino acids of expandase preferably involve replacement of:
- histidine at position 18 according to SEQ ID NO 1 by cystein, serine, threonine, asparagine, glutamine, tyrosine, lysine and arginine; more preferably serine, threonine, asparagine, glutamine, lysine and arginine; most preferably arginine;
- methionine at position 74 according to SEQ ID NO 4 by threonine or by a hydrophobic amino acid such as valine, leucine, isoleucine and phenylalanine or by an amphiphilic amino acid such as tyrosine, tryptophan, histidine, glutamine and asparagine, more preferably threonine or isoleucin;
- Threonine at position 89 according to SEQ ID NO 1 preferably by a charged amino acid, preferably a negatively charged amino acid such as aspartic acid or glutamic acid, most preferably a positively charged amino acid such as lysine, arginine or hystidine; most preferred being lysine.
- serine at position 112 according to SEQ ID NO 1 preferably by threonine;
- histidine at position 244 according to SEQ ID NO 1 by glutamine or asparagine, more preferably glutamine;
- aspartic acid at position 284 according to SEQ ID NO 1 and at position 285 in SEQ ID NO 4 by an amino acid with a polar side chain such as serine, threonine, asparagine, glutamine, glutamic acid, histidine, lysine, arginine and tyrosine, more preferably asparagine, glutamine, glutamic acid, lysine and arginine; most preferred being asparagine
- glycine at position 300 according to SEQ ID NO 1 by an amino acid with a small residue, such as alanine, serine, threonine, cystein, valine, isoleucine, leucine, asparagine and aspartic acid, more preferably valine.

Furthermore described are mutant expandases that are variants of the group consisting of the wild type expandases of S. clavuligerus, S. jumonjinensis, S. ambofaciens, S. chartreuses and N. lactamdurans. Most preferred are mutant expandases as defined hereinbefore that are variants of the wild type expandase of S. clavuligerus or N. lactamdurans. Most preferred are mutant expandases as defined hereinbefore that are variants of the wild type expandase of S. clavuligerus. Preferred mutant expandases that are mutants of the expandase of *Streptomyces clavuligerus* have modifications at least at
○ 2 or more amino acid positions selected from the group consisting of D2, T89, L277, C281 and G300.
○ 3 or more amino acid positions selected from the group consisting of D2, T89, C281, T293 and A311
○ 4 or more amino acid positions selected from the group consisting of D2, M73, T89, N90, Y217, H244, L277, E280, C281, R306, R307 and A311
○ 5 or more amino acid positions selected from the group consisting of D2, M73, T89, N90, C155, T213, R249, A278, C281, T293, R306, R307 and A311
○ 6 amino acid positions selected from the group consisting of N90+T105+G113+C281 +R306+A311.
○ 7 or more amino acid positions selected from the group consisting of D2, M73, T89, T105, G113, C155, H170, P177, D251, L277, E280, C281, T293, G300, R306, R307 and A311
○ 8 or more amino acid positions selected from the group consisting of D2, T89, N90, M99, T105, G113, C155, P177, L277, C281, R306, R307 and A311.
○ 9 amino acid positions selected from the group consisting of D2+N90+T105+G113+C155+P177+C281+R306+A311.
○ 10 amino acid positions selected from the group consisting of D2, S59, N90, T105, G113, T105, G113, C155, P177, G209, L277, C281, R306, R307, A311.
○ 11 amino acid positions selected from the group consisting of D2, N90, T105, G113, C155, P177, L277, C281, R306, R307, A311.

Preferred modifications at the respective positions are D2N, D2H, D2Y, S59G, M73H, M731, T89A, T89K, T89V, N90W, N90S, M99T, T1051, Y1 01 F, G113D, C155W, H170Y, P177L, G209A, T213A, Y217H, H244R, R249C, D251G, L277Q, L277T, A278V, E280G, C281Y, T293K, G300S, R306deletion, R307deletion, R370T and A311 D. In this notation, the letter following the number represents the amino acid (one letter code) present in the mutant expandase. The deletion at position R306 or R307 mean that the original arginine at position 306 or 307 is no longer present in the mutant expandase. Highly preferred mutant expandases are the mutant expandases that are summarized in Table 3.

Preferably, the mutant expandases described herein have an improved expandase activity on adipyl-6-APA as defined herein before, the mutant expandases provided by the present invention have likewise, an improved expandase activity on Pen-G. Preferably, the mutant expandases have an improved expandase on both adipyl-6-APA as well as Pen-G. Mutant expandases with an improved activity on Pen-G can be used advantageously in a process for the production of phenylacetyl-7-ADCA as described further below.

The present invention also provides mutant expandases with a decreased or even absent expandase activity with iso-penicillin N (iPN). Preferably the expandase activity with either adipyl-6-APA or Pen-G is not affected, but more preferably the expandase activity with either adipyl-6-APA or Pen-G or both is improved as defined hereinbefore. The advantage of these more preferred mutant expandases is that the decreased or even absent expandase activity with iso-penicillin N (iPN) results in less byproduct in a fermentation process to produce ad-7-ADCA or phenylacetyl-7-ADCA.

Preferred mutant expandases have a decreased or even absent expandase activity with iso-penicillin N (iPN) as a substrate optionally combined with an improved expandase activity on ad-6-APA as a substrate and have been modified at position 89 according to the amino acid numbering of SEQ ID No 1. whereby the naturally occurring amino acid has been replaced by lysine.

A highly preferred mutant expandase is selected from the group consisting of H101, H106, H111, H122, H127, H262, H301, H305, H308, H309, H401, H402, H403, H501, H502, H503, H504, H505, H506, H507, H508, H601, H602, H603, H604, H605, H606, H607, H608, H609, H650, H651, H652, H653, H654, H655, H656, H657, H658, H659, H660, H661, H662, G601, G602, G603, G604, G605, G606, G607, G608, G609, G610, G611, G613 and G614 (see Table 3).

In a second aspect, the invention provides a polynucleotide encoding the mutant expandase of the present invention. The polynucleotide encoding the mutant expandase according to the present invention can be any polynucleotide that encodes the proper amino acid sequence according to the invention. This implies that it may correspond to the *cefE* gene of *S*. *clavuligerus* except for the nucleotides encoding the modifications at the respective amino acid positions. Alternatively, the polynucleotide of the invention may comprise a coding sequence in which the codon usage for the various amino acids deviates from the codon usage in *S*. *clavuligerus* and/or in *N. lactamdurans.* For example, the codon usage may be adapted to the codon usage of a particular host cell, which will or has been transformed with the DNA fragment encoding the altered expandase.

In a third aspect, the invention provides an expression vector or expression cassette comprising the polynucleotide of the invention as defined hereinbefore.

In a fourth aspect, the invention provides a transformed host cell, transformed with the polynucleotide of the invention or the expression vector or expression cassette of the invention. The transformed host cell may be used for the production of the mutant expandase of the invention or the host cell may be used for the production of a beta-lactam compound of interest.

Host cells for the production of the mutant expandase of the invention are preferably host cells which are known in the art for their efficient protein or enzyme production, either extracellular or intracellularly, for example microorganisms such as fungi, yeast and bacteria. Examples of preferred host cells comprise, but are not limited to, the following genera: *Aspergillus* (e.g. *A. niger, A. oryzea*), *Penicillium* (e.g. *P*. *emersonii, P. chrysogenum*), *Saccharomyces* (e.g. *S*. *cerevisiae*), *Kluyveromyces* (e.g. *K. lactis*), *Bacillus* (e.g. *B. subtilis, B. licheniformis, B. amyloliquefaciens). Escherichia, (E. coli*), Streptomyces (e.g. *S. clavuligerus*).

Host cells for the production of a beta-lactam compound of interest are preferably host cells that are known in the art for their efficient beta-lactam compound production. Examples of preferred host cells comprise, but are not limited to, to the following genera: *Penicillium* (e.g. *P. chrysogenum), Acremonium (e.g. A. chrysogenum), Streptomyces (e.g. S. clavuligerus), Nocardia (e.g. N. lactamdurans), Lysobacter (e.g. L. lactamgenus)* and *Flavobacterium* species.

In a fifth aspect, the invention provides a process for the production of the mutant expandase of the invention comprising cultivating the transformed host cell according to the invention under conditions conducive to the production of the mutant expandase and, optionally, recovering the mutant expandase. The recovered mutant expandase may be used advantageously in an in vitro process to produce a desired cephalosporin from a corresponding penicillin, for instance the recovered mutant expandase may be used in a process to produce phenylacetyl-7-ADCA from Pen-G or adipyl-7-ADCA from adipyl -6-APA.

In a sixth aspect, the invention provides a process for the production of a beta-lactam compound of interest comprising cultivating the transformed host cell according to the invention under conditions conducive to the production of the beta-lactam compound of interest and, optionally, recovering the beta-lactam compound. Preferred beta-lactam compounds belong to the group of cephalosporins such as phenylacetyl-7-ADCA, adipyl-7-ADCA, adipyl-7-ADAC and adipyl-7-ACA. In a preferred embodiment, the invention provides a process for the production of phenylacetyl-7-ADCA or adipyl-7-ADCA by cultivating a selected strain of *Penicillium chrysogenum,* that has been transformed with a selected polynucleotide of the invention that encodes a mutant expandase of the invention. For the production of phenylacetyl-7-ADCA, a mutant expandase is selected that has a high improvement factor on Pen-G as a substrate. For the production of adipyl-7-ADCA, a mutant expandase is selected that has a high improvement factor on ad-6-APA as a substrate.

In another preferred embodiment, the invention provides a process for the production of 7-ADCA comprising the process for the production of phenylacetyl-7-ADCA or adipyl-7-ADCA as described herein before, followed by, after optional purification of said 7-ADCA-derivatives, a process step in which the phenylacetyl or adipyl side chain of phenylacetyl-7-ADCA and adipyl-7-ADCA respectively is cleaved off thereby generating 7-ADCA and the liberated side chains acid. Said cleavage can be obtained by chemical means or, more preferably, enzymatically using an acylase enzyme. Suitable acylases for the cleavage of the adipyl side chain are obtainable from various *Pseudomonas* species such as *Pseudomonas* SY-77 or *Pseudomonas* SE-83. Suitable acylases fro the cleavage of the phenylacetyl side chains are the penicillin acylases from *Escherichia coli* or *Alcaligenes feacalis.*

In a seventh aspect, the invention provides a process for the production of a cephalosporin from a corresponding penicillin whereby the expansion of the 5-membered thiazolidine ring of the penicillin to the 6-membered dihydrothiazine ring of the cephalosporin occurs in an *in vitro* process, catalyzed by a mutant expandase of the invention. In a preferred process, adipyl-6-APA is expanded to the corresponding adipyl-7-ADCA by a mutant expandase of the invention, preferably a mutant expandase that has a high improvement factor adipyl-6-APA as a substrate. In another preferred process, Pen-G is expanded to the corresponding phenylacetyl-7-ADCA by a mutant expandase of the invention, preferably a mutant expandase that has a high improvement factor on Pen-G as a substrate.

The process may be followed by, after optional purification of said 7-ADCA-derivatives, a process step in which the side chains of adipyl-7-ADCA and phenylacetyl-7-ADCA are cleaved off thereby generating 7-ADCA and the liberated side chains acid - supra vide.

The desired end product 7-ADCA can be recovered according to methods known in the art involving chemical or enzymatic cleavage of the side chain of adipyl-7-ADCA or phenylacetyl-7-ADCA and optionally the resulting 7-ADCA can be further purified and/or crystallized.

Cloning of the gene encoding a model polypeptide with expandase activity can be carried out according to methods known in the art.

Preferred model polypeptides with expandase activity are selected from the group consisting of a polypeptide with expandase activity obtainable from *Streptomyces clavuligerus,* preferably having an amino acid sequence according to SEQ ID NO: 1 and polypeptides with expandase activity having an amino acid sequence with a percentage identity with SEQ ID NO: 1 of at least 70%, preferably at least 75%, more preferably at least 80%, more preferably at least 85%, more preferably at least 90%, most preferably at least 95%, such as the expandase enzymes that are summarized in Table 1.

Any mutagenesis technique can be employed which results in mutations over the entire gene. Suitable techniques are error prone (EP) PCR (Polymerase Chain Reaction) and/or by using saturated Mutation Primer PCR (sMPP) exactly according to WO 03/010183.

### Materials and Methods

### 1. General

Oligonucleotides were synthesized by Invitrogen (Carlsbad CA, US).

DNA sequencing was carried out by SEQLAB (Göttingen, Germany) or by Baseclear (Leiden, The Netherlands).

Restriction enzymes were purchased from Invitrogen.

The protein assays were carried out according to the method described by Bradford, M.M. (1976) Anal Biochem. 72:248-54

*Escherichia coli* DH1 0B electromax competent cells were obtained from Invitrogen. The protocol was delivered by the manufacturer.

SDS-PAGE electrophoresis was carried out in the system supplied by Invitrogen. NuPAGE Novex Bis-Tris Gels (Invitrogen). SimplyBlue SafeStain Microwave protocol

### Overview of used constructs

| Plasmid | Description |
|---|---|
| PBAD-MH-Zeo-MBP-ScEwt | Arabinose inducible *E.coli* expression construct with maltose binding protein (MBP) fused to wild type *S.clavuligerus* expandase. Zeocine marker |
| PBAD-MH-Zeo-MBP-H 127 | Idem but fused to H127 expandase (see also Table 3x) |
| PBAD-MH-Zeo-MBP-H406 | Idem but fused to H406 expandase (see also Table 3x) |
| PBAD-MH-Zeo-MBP-H402 | Idem but fused to H402 expandase (see also Table 3x) |
| PBAD-MH-Zeo-MBP-H403 | Idem but fused to H403 expandase (see also Table 3x) |

### 1. Cloning of the E. coli expression vectors

The fusion product is ligated in the pBAD/MH MBP-ScEwt Dest zeo vector by an *EcoRI*/*Sal*I digestion. This replaces the wild type expandase gene by the library genes. To prevent significant numbers of wild type constructs, the ligation constructs were digested with SmaI.

### 2. Construction of the libraries

Transformation of *E. coli w*ith the ligation mix yielded libraries of roughly 12,000-13,000 colonies. To test the quality of the libraries, a random set of colonies was selected and the constructs were digested with restriction enzymes. This showed that approximately 26% of the library revealed an aberrant pattern. Next, sequence analysis was performed on 10 regular clones to determine mutation frequencies. Sequences showed that both saturation and error prone mutations were present at a satisfactory level.

### 3. Expression and screening of mutant expandases

### 3.1. Screening of the cefE library for improved activity

*E. coli* TG1 cells containing the library A were plated and grown on agar plates. Colonies are picked robotically into 96-wells microtiter plates containing liquid medium (2YT, 30 µg/ml chloramphenicol, 05% glucose), and grown to saturation by shaking at 37°C. Cells are subcultured in 96 well megatiter plates; 1:40 inoculum into 2YT, 30 µg/ml chloramphenicol and induced by 0.2 mM IPTG shaking at 28°C for 24 h. Cells are pelleted and washed with an equal volume of buffer (5 mM morpholine pH7.5). After spinning, the cells are resuspended in lysis mix for primary screening Expandase assay (1)).

### 3.2. Primary screening in microtitre plates (MTP)

### Growth and induction

The expandase *E. coli* library is plated on Luria-Bertani (LB) medium plates low salt agar + 25 µg/ml Zeocine and incubated overnight at 37°C. Microtitre plates (MTP) with 150 µl LB low salt medium and 25 µg/ml Zeocine are inoculated from the plates and incubated 36 hrs at 25°C and 550 rpm.

From the MTP, 5 µl is inoculated in deep well plate with 1 ml 2*TY (tryptone and yeast extract) medium + 50 µg/ml Zeocine + arabinose. To the remaining culture in the MTP, 100 µl 50% glycerol is added and frozen at -80 °C as glycerol stock.

The deep well plate is incubated for 30 hrs at 25°C and 550 rpm. The deep well plate cultures are centrifuged at 2750 rpm (Heraeus multifuge 4 kr.) for 10 min. The supernatant is discarded and the pellet is frozen at -20 °C.

### Cell free extract (CFE)

The frozen pellets obtained in the previous section are thawed in 200µl extraction buffer (50 mM Tris/HCl pH=7.5; 5 mM DTT; 0.1 mg/ml DNAse; 5 mM MgSO₄·H₂O and 0.5 mg/ml lysozyme). To resuspend pellets, the plates are shaken. The plates are incubated for 30 minutes at room temperature and centrifuged for 10 minutes at 2750 rpm.

### 3.3 Secondary screening in shake flasks

### Growth and induction

Selected colonies are inoculated from plate in 10 ml LB low salt + Zeocin 25 µg/ml and incubated overnight at 37°C, 280 rpm. The grown culture is inoculated in 100 ml LB low salt with 25 µg/ml Zeocine at an optical density (600 nm) between 0.010 and 0.050 (Biochrom Ultraspec 2000). After growth at 37°C, 280 rpm, the cells are harvested at an optical density at 600 nm between 0.4-0.6. Then, arabinose is added (final concentration 0.2%) and induced overnight at 27°C at 220 rpm.

### 4. Expandase assays

### 4.1 Expandase assay (1) - Screening assay (primary assay):

Expandase assay buffer: 5 mM morpholine buffer pH 7,5 and cofactor mix (final concentrations 50 µM ATP, 1 mM DTT, 60 µM FeSO4, 2,7 mM ascorbate), 500 µM ad-6-APA, 1/10^{th} volume of *E*. *coli cell* lysate and 2.4 mM α-KG. Each cofactor was made in buffer pH 7.5, which was critical for activity.

The expandase reaction was found to be linear for up to 3 hours with respect to adipyl-7ADCA formation/detection. The optimal temperature for the in vitro expandase reaction was determined to be approximately 11°C, probably due to instability of the enzyme or production at higher temperatures. Nevertheless, actual screening was performed at a temperature between room temperature and 25°C, which is more reflective of the desired reaction condition temperature.

Screening of the family shuffled libraries for improved 7-ADCA formation was determined by Flow Injection Analysis-MS (FIA-MS, negative mode, Mass Transition [M-H]⁻341>295). Final analysis: LC column: using solvent conditions between 0.1% formic acid in 40% MeOH to 100% MeOH. Two channels (two mass transitions [M-H⁻]) are used to monitor for each reaction product. Samples are analyzed after 30 minutes of reaction.

### 4.2 Expandase assay (2) - Secondary expandase assay

Expandase assay buffer: 5 mM morpholine buffer pH 7,5 and cofactor mix (final concentrations: 50 µM ATP, 1 mM DTT, 60 µM FeSO4, 2,7 mM ascorbate), 2 mM adipyl-6-APA, 1/10^{th} volume of *E. coli* cell lysate and 2.4 mM α-ketoglutarate. Each cofactor was made in buffer pH 7.5, which was critical for activity. The conversion was performed at 29 °C and samples were taken between 0 and 30 minutes. The reaction was stopped with 60% (v/v) acetonitrile final concentration. The formed adipyl-7-ADCA was analysed on HPLC. (Mobile phase: 10% acetonitrile in 10 mM potassium phosphate buffer pH 3.0) Column: X-Terra™ RP18 3.5µ. 3.9x100 mm detected at 214 and 254 nm (254 nm to analyse expanded antibiotics). HPLC data analysis: software: Waters Millennium^{32®} 3.20.

### 4.3 Expandase assay (3) - Expandase assay

A total of 300 µl of a reaction mixture consisting of 50 mM Tris/HCl pH 7.5; 1 mM DTT; 2.7 mM Ascorbate; 0.05 mM ATP; 24 mM α-ketoglutarate; 0.06 mM FeSO₄.7H₂O; 4 mM adipyl-6-amino-penicillanic acid (ad-6-APA) was added to 75 µl CFE and incubated at 29°C for the desired time. Adding 60 µl maleic acid with 10 g/l EDTA stopped the reaction. The formation of adipyl-7-amino-desacetoxy-cephalosporinic acid (ad-7-ADCA) was detected using ¹H-NMR.

The adipyl-6-APA was obtained from 6-APA and adipic acid catalyzed by penicillin acylase (EC 3.5.1.11). Alternatively, adipyl-6-APA can be obtained by culturing for instance a suitable *Penicillium chrysogenum* strain in the presence of adipic acid as precursor.

### EXAMPLES

### Example 1

### Cloning of wild type expandase

The *cefE* gene was PCR cloned from *S*. *clavuligerus* genomic DNA and ligated in GFP-fusion vectors, the so-called pCK series, and used to transform *E*. *coli* TG1 (Amersham Pharmacia Biotech, Inc. NJ USA). Several colonies from the plated transformations were selected and checked for an active clone. Several active clones were identified. Clone '778' appeared to be a wild type gene but interestingly, one other clone, clone "779", revealed strongly improved activity on adipyl-6-APA expansion. The product of clone "779" was sequenced and revealed three amino acid mutations (H18R, D284N and G300V). The adipyl-7-ADCA production of the clones analysed by Flow Injection Analysis-Mass Spectrometry (FIA/MS) showed that the Sc-*cefE* mutant "779" was superior to the wild-type parents. Measurement of GFP fluorescence suggests that the improvement of the expandase enzyme product of clone "779" is not due to improved expression of *cefE* therein. Hence, it was concluded that the improvement of the expandase enzyme product of clone "779" is an improvement of the activity as a result of the amino acid substitutions.

### pCK derived GFP fusion vectors

Fusion tag vector series (called pCK series) containing a His tag fused to the 3' end of expandase with green fluorescent protein (GFP) fusion at the 3' end of the HIS tag were constructed. The GFP containing vectors enable high throughput normalisation of expandase activity data in subsequent assays with respect to protein concentration. The vectors contain the p15A origin of replication (low copy number), and a *lac* promoter that is repressed in the presence of glucose.

### Example 2

### Family shuffling and Screening of wild-type N. lactamdurans and S. clavuligerus cefE genes - Library A

The expandase genes were shuffled according to the process described in EP752008. Expandase mutant libraries were made using the GFP-fusion tag vectors (pCK). Only the *Cef*E ORF was shuffled, while other sequences where not changed. After moving the shuffled pool of expandases into the pCK vectors, one library set (Library A) was made using the two parental wild type expandase genes (*S*. *clavuligerus* and *N. lactamdurans)* plus clone "779".

Initial plating of a sample of the library showed that the libraries contained at least 70% green fluorescent colonies. Due to this, it was not considered necessary to pre-screen out non-fluorescent colonies before proceeding to the first FIA/MS screens.

DNA sequencing of randomly picked colonies form library set A indicated that all parents were represented in the library, so this library was selected for initial screening.

720 clones from library A were screened and 14 clones (mutant expandases H101, H102, H104, H106, H108, H111, H112, H114, H115, H117, H120, H122, H125, H127) were retested and the preliminary results showed an expandase activity improvement compared to the *S*. *clavuligerus* expandase (clone "778") as judged by *in vitro* expandase assay (1). These clones were chosen for further testing. Normalization of the expandase activity with respect to expandase-GFP concentration (i.e. correcting for differences in the expandase protein concentration) confirmed that these clones were improved in specific activity.

### Example 3

### Family shuffling of wild-type N. lactamdurans and S. clavuligerus cefE genes. Library B

Library B was constructed shuffling wild type *N. lactamdurans* and *S*. *clavuligerus cefE* as parent genes. The "779" hit sequence was not used in construction of this library. This expandase library showed to have a significant level of inactive or very poorly active clones in the library. In order to remove inactive clones from further testing, a FACS-based pre-screen was used for the library B screening to improve the percentage of active clones that enter the expandase assays.
After FACS sorting based on their fluorescence by GFP, selected cells were recovered under repressing conditions. DNA prepared from these cultures was used to transform *E. coli* TG1. These expandase constructs used are also fusion proteins with GFP as described for library A.

70-80 percent of the library was GFP-active. In total, 2200 clones from this library were then screened using the *in vitro* expandase assay and FIA-MS detection of adipyl-7-ADCA formation.

Fourteen possible hits were selected (H200 series). Four of these hits, amongst them H262, were confirmed through retesting procedure including retransformation and *in vitro* testing and analysis MS.

### Example 4

### Construction of chimeric expandase sequences using shuffled expandases Library C

Using traditional molecular biological techniques and bioinformatics tools, the hit sequences from the two above described libraries (libraries A and B) were recombined, resulting in library C (H300 series). Three resulting chimeras were tested for activity, amongst them expandase H301.

Six remaining clones (amongst them: H305, H307, H308, H309) were not tested as GFP fusion product nor analysed by FIA-MS. These clones were directly recloned in the pSJ vector series fused with maltose binding protein (MBP) upstream of the Ce/E mutant genes and analysed, after the *In vitro* assay, on HPLC.

### Example 5

### Recloning of genes encoding improved expandases using pSJ based vector series and enzymatic activity of the improved expandases

pSJ08 was used as cloning vector for the expandase libraries. This plasmid was constructed by replacing the expandase gene (as *Ndel-Nsil* fragment) by a small oligonucleotide introducing a *Notl* restriction site. *S*. *clavuligerus cefE* was cloned as *Nde*I*-Nsi*I fragment in pSJ08, resulting in pSJ05.

pSJ05 contained an additional *Nde*I site just upstream of the *Nsi*I site. This site was removed to prevent possible interference with further cloning of the shuffled genes in the *Nde*I*-Nsi*I sites, resulting in pSJ11. Next, maltose binding protein (MBP) was fused to the 5' end of *cef*E resulting in pSJ10. A plasmid containing MBP-*cefE* fusion genes for *Nocardia lactambdurans* (pSJ01) was also constructed. DNA sequence analysis confirmed all sequences.

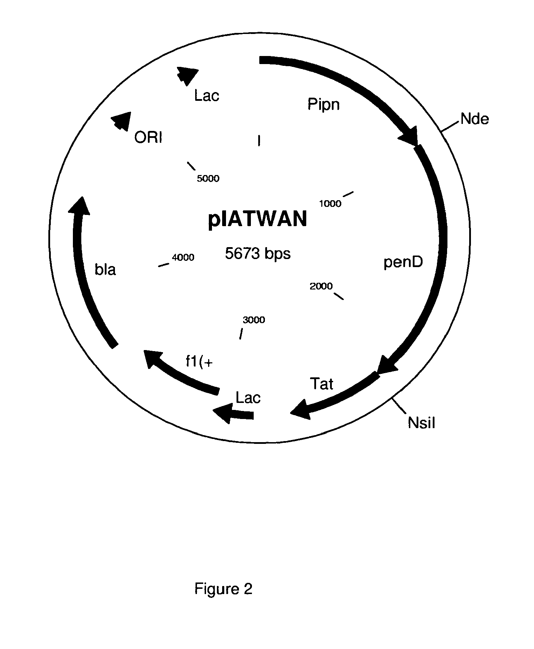

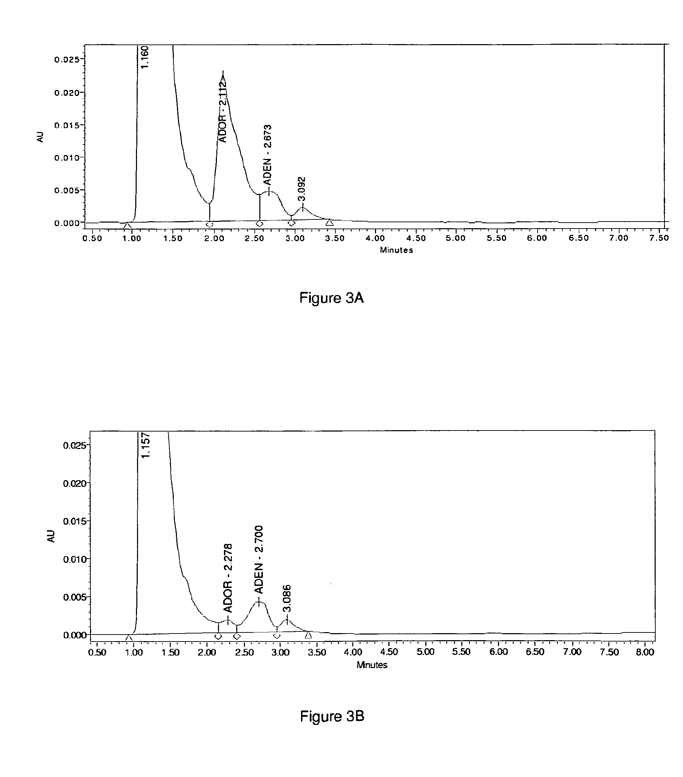

Improved clones from the A, B and C library were recloned from the pCK-vectors into the pSJ10 vector (MBP-cefE behind the tac promoter). This was done by amplifying the improved expandases by PCR. The forward (5'-end) primer introduces a *Nde*I and the reverse (3'-end) primer introduces a *Nsi*I site. After digestion with *Nde*I and *Nsi*I the hit was cloned in the *E*. *coli* vector (pSJ10) and in the *Penicillium chrysogenum* vector (pIATWAn- Figure 2/3) Table 2.

Finally, a few clones of each were sequenced and one of each, containing the verified correct sequence, was used for further analysis.

The DNA and amino acid sequences of the best expandase hits are included in the sequence listing - see Table 2 for an overview.

### Induction of expression

*E. coli* NM554 was grown overnight on 2xTY medium with chloramphenicol (30 µg/ml) at 37°C. The cells were diluted to an optical density at 540 nm (OD₅₄₀) of 0.010-0.020 in fresh 2xTY with chloramphenicol (30 µg/ml). When cells reached an optical density at 540 nm of 0.4-0.6, IPTG was added (final concentration 0.5 mM) and expression was induced overnight at 27°C.

The cells were washed and resuspended in extraction buffer (5 mM DTT, 50 mM Tris/HCl pH 7.5 with lysozym) and sonicated (on ice water). After centrifugation the supernatant is used for the expandase assay (2).

Expression of the expandase in the cell free extract was determined by SDS-PAGE.

**Table 2. H100, H200 and H300 series expandase hits, their mutation(s). their E. coli and Penicillium expression vectors and their expandase activity (assay 2).**

| Expandase | SEQ ID NO | backbone sequence | mutations | Vector | | Spec. act.* | I.F. (**) |
|---|---|---|---|---|---|---|---|
| | | | | *Penicillium* | MBP-cefE | | |
| *Cef*E of S. clavuligerus "778" | 1 | wild type | none | | pAJL104 | 12 | 1 |
| *Cef*E of N. lactamdurans | 2 | wild type | none | | pJS01 | 4 | 0,3 |
| "779" | 3 | 778 | H18R+D284N +G300V | | | | |
| H101 | 6 | | | | pSJ101 | 63 | 5,3 |
| H106 | | 778 | H18R, S112T | | pSJ106 | 59 | 4,9 |
| H111 | 7 | | | | pSJ111 | 77 | 6,4 |
| H122 | 8 | | | | pSJ122 | 61 | 5,1 |
| H127 | 5 | | | | pSJ127 | 72 | 6,0 |
| H262 | 4 | | Shuffled | plAT262 | pSJ262 | 40 | 3,3 |
| H301 | 9 | | H18R, D284N, G300V, M73T | plAT301 | pSJ301 | 49 | 4,1 |
| H305 | 10 | H261 | G300V | plAT305 | pSJ305 | 45 | 3,8 |
| H307 | 11 | H262 | M74T | plAT307 | pSJ307 | | |
| H308 | 12 | H262 | D285N | plAT308 | pSJ308 | 48 | 4,0 |
| H309 | 13 | H262 | M74T,D285N | plAT309 | pSJ309 | 46 | 3,8 |

| | | | | | | | |
|---|---|---|---|---|---|---|---|
| * Specific activity (nmol 7-ADCA.min⁻¹.mg⁻¹ protein) ** I.F. = Improvement factor; equal the ratio of the specific activity of the mutant expandase and the specific activity of the expandase from *S*. *clavuligeris* | | | | | | | |

### Example 6

### Expression of expandase H122 and H127 in Penicillium chrysogenum

Expandase variants H122 and H127 were cloned into vectors for *Penicillium chrysogenum* expression (plAT series plasmids, Table 2). Shuffled expandase genes cloned into a plasmid vector with an *E*. *coli* replicon and selectable marker. A promoter compatible with expression in *P. chrysogenum, i.e.,* the IPNS promoter, was used to express the expandase gene. Additionally a selectable marker for *Penicillium chrysogenum* is present (amdS).

The expression constructs were obtained by digesting the expression construct, plAT127 (expandase H127) and plant122 (expandase H122), with *Notl.* A high producing *Penicillium chrysogenum* is co-transformed with the linear fragments together with the amdS expression cassette (*Hind*III digest).

By precursing with adipate, adipyl -7-ADCA production can be tested in strains expressing active expandase genes.

The cephalosporin production of the strain expression expandase H127 was compared to the strain expressing wild type expandase in batch regime with lactose as carbon source. The adipyl-7-ADCA production is increased between 10-50% and the total β-lactam production was increased by 0-30%, depending on the lactose and the adipate concentration.

Additionally, expandase H122 was expressed in *Penicillium chrysogenum.* Comparison of the cephalosporin production with the strain expressing wild type expandase showed that the adipyl -7-ADCA production is increased between 0 to 50% and the total beta-lactam production varied between -20 to 0% depending on the lactose and the adipate concentration in the media.

### Example 7

### 1^{st} generation improved expandases (H400 series).

The first generation expandase library was constructed by performing error prone (EP) PCR (Polymerase Chain Reaction) on wild type *Streptomyces clavuligerus* CefE gene (SEQ ID No.1). After screening this library for improved conversion of ad-6-APA to ad-7-ADCA, 3 different mutant genes were selected. The mutants exhibited an improved ad-6-APA expansion activity up to 2.5-fold (H401, H402, H403: see Table 3).

### Example 8

### 2^{nd} generation improved expandases (H500 series)

The second-generation expandase library was constructed by using saturated Mutation Primer PCR (sMPP) exactly according to WO 03/010183. The selected mutant genes from the first generation (H401, H402 and H403) were used as templates for the construction of the 2^{nd} generation library.

The sMPP was performed by using Taq polymerase, thereby introducing additional mutations (random) and increasing the variation of the library. Designed primers annealed at the mutation positions and were saturated at the mutations found in the 1^{s1} generation expandase hits. Additionally, a universal forward and reverse primer was designed in which an *Nde*I and *Nsi*I site were introduced respectively. This facilitated cloning into *Penicillium* expression vector for testing of the mutant expandases *in vivo.*

The library was grown and expression of the expandase was induced as described in the materials and methods section. The formation of ad-7-ADCA was determined using NMR as described in the materials and methods section.

Approximately 150 improved mutant expandases were selected and retested for their ad-6-APA expandase activity. Based upon the results of this retest, 17 mutants were selected for final tests in shake flasks and analysis by HPLC as described in the Materials and Methods.

In total, 15 of the 17 selected hits showed a significant improved expandase activity with ad-6-APA as a substrate. To exclude the false positive selection of expression mutants, protein levels were quantified on SDS-PAGE. This confirmed that the improved activity was not due to stronger expression of expandase in *E*. *coli,* but that the improvement could be attributed to an improved specific activity of the expandase mutant.

In total, 8 mutant expandases were identified which exhibited an improvement factor of their ad-6-APA expandase up to 4,5-fold (compared to the wild type expandase of S. clavuligerus - SEQ ID No 1.). These mutants are designated H501 - H508 (see Table 3).

### Example 9

### 3^{rd} generation of improved expandases (H600 series)

The 8 mutants obtained in the 2^{nd} generation Example 2 (H501 - H508) were used as templates for the construction of the 3^{rd} generation expandase library. This library was constructed in the same way as the 2^{nd} generation expandase library. Additionally, the SKA signaling sequence at the C-terminus of the expandases was changed to SKD, causing expression of expandase solely in the cytosol when expressed in *Penicillium.*

The screening of this library yielded 22 different expandase mutant genes that were significantly improved between 5-fold and 11-fold compared to *S*. *clavuligerus* expandase (Table 3, H600 series).

Screening of the same library for a second time yielded 13 additional mutant expandases (G601 - G611 and G613 - G614; see Table 3)

### Example 10

### Activity of improved mutant expandases with iso-penicillin N (iPN) and Penicillin-G (Pen-G) as a substrate.

The activity of several mutant expandases was measured using iPN and Pen-G as a substrate. The assay with iPN as a substrate was carried out as described in the materials and methods section except that ad-6-APA was replaced by the same concentration of iPN (4 mM). The assay with Pen-G as a substrate was carried out as described in the materials and methods section except that ad-6-APA was replaced by 7 mM Pen-G. Table 3 summarizes the activities of the various expandase mutants for iPN and Pen-G.

Whereas most of the expandase mutants tested exhibited a 5-fold improvement of their expandase activity with iPN, mutants that carry the T89K mutation lost virtually all activity towards iPN.

The activity towards Pen-G of the various expandase mutants tested was either the same as the wild type expandase (improvement factor is 1) or improved up to 8-fold. The data further show that there is no correlation whatsoever between the improvement factors obtained for a single mutant with ad-6-APA and Pen-G as a substrate. The ratio between the respective improvement factors for ad-6-APA and Pen-G vary in a range from 0.1 (e.g. H654) to 1.2 (e.g. H503).

**Table 3. Mutant expandases and their expandase activity toward ad-6-APA, Pen-G and iPN. Each mutant is identified by its code; the mutations are introduced in the model expandase from S. clavuligerus (SEQ ID NO 1). The expandase activity is expressed as improvement factor compared to the model expandase from S. clavuligerus (expandase activity = 1 by definition) according to the in vitro expandase assays described in the Materials and Methods and Example 4.**

| Mutant | | Mutations | Expandase Activity (Improvement factor) | | |
|---|---|---|---|---|---|
| | Code | | ad-6-APA | Pen-G | iPN |
| 1. | H401 | L277Q | 1.5 | | |
| 2. | H402 | D2N+C281Y | 2.5 | | |
| 3. | H403 | T89A | 1.5 | | |
| 4. | H501 | T89A+C281Y | 3.9 | 3.0 | 2.6 |
| 5. | H502 | D2Y+C281Y+R306?+A311D | 4.5 | 4.0 | 5.3 |
| 6. | H503 | D2N+T89V+C281Y | 3.5 | 4.0 | 3.2 |
| 7. | H504 | T89K+C281 Y+T293K | 3.9 | 1.0 | 0.0 |
| 8. | H505 | T89K+C281Y | 3.3 | 1.0 | 0.0 |
| 9. | H506 | L277Q+G300S | 2.9 | 3.0 | 4.6 |
| 10. | H507 | D2Y+N90S+T1051+G113D+C155W+P177L+L277T+C281Y | 3.7 | 3.0 | 3.0 |
| 11. | H508 | D2H+L277Q+E280G+C281Y | 4.6 | 4.0 | 5.1 |
| 12. | H601 | M73H+T89K+C281Y+A311D | 7.5 | 1.2 | 0.0 |
| 13. | H602 | T89K+Y217H+C281Y+A311D | 7.6 | 1.1 | 0.0 |
| 14. | H603 | T89K+H244R+C281Y+A311D | 6.8 | 1.7 | 0.0 |
| 15. | H604 | T89K+C281Y+R307T+A311D | 6.9 | 1.4 | 0.0 |
| 16. | H605 | T89K+C281Y+R307T+A311D | 7.2 | 1.5 | 0.0 |
| 17. | H606 | M731+T89K+C281 Y+T293K+G300S+R307T+A311D | 8.8 | 1.9 | 0.0 |
| 18. | H607 | T89K+C281Y+T293K+G300S+A311D | 7.0 | 1.3 | 0.0 |
| 19. | H608 | T89K+C281Y+A311D | 5.8 | 1.0 | 0.0 |
| 20. | H609 | M73H+T89K+T213A+C281 Y+A311D | 6.2 | 1.3 | 0.0 |
| 21. | H650 | T1051+G113D+C155W+P177L+C281Y+R306?+A311D | 8.2 | 7.1 | 11,0 |
| 22. | H651 | C155W+P177L+L277Q+E280G+C281Y+R306?+A311D | 10.6 | 8.9 | 11,0 |
| 23. | H652 | L277Q+C281 Y+R306?+A311D | | 5.0 | 10,0 |
| 24. | H653 | T89V+C281Y+R306?+A311D | 9.0 | 5.8 | 11,0 |
| 25. | H654 | T89K+R249C+C281Y+R306?+A311D | 6.3 | 0.8 | 0,0 |
| 26. | H655 | D2Y+N90S+T1051+G113D+C155W+P177L+C281Y+R306?+ A311D | 8.2 | 7.1 | 10,0 |
| 27. | H656 | D2N+N90S+M99T+T1051+G113D+C281Y+R306?+A311D | 5.0 | 3.7 | 5,0 |
| 28. | H657 | N90S+T105I+G113D+C281 Y+R306?+A311D | 6.9 | 4.6 | 8,0 |
| 29. | H658 | D2N+T89V+C281Y+R306?+A311D | 8.4 | 6.3 | 10,0 |
| 30. | H659 | T89K+C281Y+R306?+A311D | 6.4 | 0.8 | 0,0 |
| 31. | H660 | N90W+C281Y+R306?+A311D | 5.3 | 4.4 | 5,0 |
| 32. | H661 | D2Y+C155W+C281 Y+R306?+A311D | 9.7 | 6.4 | |
| 33. | H662 | D2Y+N90S+T1051+G113D+C155W+P177L+L277T+C281Y+ R306?+A311D | 5.6 | 5.2 | 6,0 |
| 34. | G601 | T89V+A278V+C281L+307deletion+A311D | 7.4 | 6.8 | |
| 35. | G602 | D2N+T89V+C155W+P177L+L277T+C281Y+A311D | 3.9 | 4.4 | |
| 36. | G603 | S59G+N90S+T1051+G113D+C155W+P177L+G209S+C281 Y+307deletion+A311D | 6.3 | 6.4 | |
| 37. | G604 | D2N+N90W+C281 Y+307deletion+A311D | 4.6 | 5.7 | |
| 38. | G605 | D2Y+C155W+P1771+C281Y+307deletion+A311D+SGRS | 5.1 | 4.6 | |
| 39. | G606 | D2Y+N90S+Y101F+T1051+G113D+C155W+P1771+C281Y+ 307deletion+A311D | 8.2 | 8.1 | |
| 40. | G607 | N90S+T105I+G113D+C155W+P1771+C281Y+R307T+A311D | 7.2 | 6.7 | |
| 41. | G608 | D2Y+N90S+T1051+G113D+C155W+P1771+L277T+C281Y+ R3071+A311D | 6.0 | 4.6 | |
| 42. | G609 | D2Y+T89V+C281 Y+307deletion+A311D | 6.6 | 4.5 | |
| 43. | G610 | D2N+T89V+T1051+G13D+C155W+P177L+C281Y+A311D | 6.4 | 7.7 | |
| 44. | G611 | T89V+T1051+G13D+C155W+P177L+C281 Q+307deletion+A 311D | 7.0 | 7.9 | |
| 45. | G613 | T89V+H170Y+D251 G+L277Q+C281 Y+307deletion+A311D | 4.3 | 4.4 | |
| 46. | G614 | D2Y+N90S+T1051+G113D+C155w+P177L+L277T+C281Y+ T293K+307deletion+A311D | 7.2 | 8.9 | |

Table 3 shows that mutant expandases have been obtained which have improvement factors in the range of 1.5 to 10.6-fold.

## Claims

1. A mutant expandase that is a variant of a model polypeptide with expandase activity whereby threonine at amino acid position 89 in the model peptide has been replaced by lysine using the amino acid position numbering of the amino acid sequence of the expandase enzyme as depicted in SEQ ID NO: 1 and whereby the model polypeptide with expandase activity is having the amino acid sequence as depicted in SEQ ID NO: 1 or an amino acid sequence having at least 70% identity with SEQ ID NO: 1.

2. A mutant expandase according to claim 1 whereby the mutant expandase has been modified at amino acid position 89 as described in claim 1 and has been modified at at least an amino acid position selected from group 1 consisting of positions 2, 18, 59, 73, 74, 90, 99, 101, 105, 112, 113, 155, 170, 177, 209, 213, 217, 244, 249, 251, 277, 278, 280, 281, 284, 293, 300, 307 and 311 using the amino acid position numbering of the amino acid sequence of the expandase enzyme encoded by the cefE gene of *Streptomyces clavuligerus* (SEQ ID NO: 1).

3. A mutant expandase according to claim 1 whereby the mutant expandase has been modified at amino acid position 89 as described in claim 1 and has been modified at at least an amino acid position selected from the group 2 consisting of positions 2, 18, 59, 90, 99, 101, 105, 112, 113, 170, 177, 209, 213, 217, 249, 251, 278, 280, 284 and 293, optionally in combination with at least an amino acid position selected from the group 1 and which does not belong to group 2.

4. A mutant expandase according to claim 1 and which is selected from the group consisting of:
T89K+C281Y+T293K
T89K+C281Y
M73H+T89K+C281Y+A311D
T89K+Y217H+C281Y+A311D
T89K+H244R+C281Y+A311D
T89K+C281Y+R307T+A311D
T89K+C281Y+R307T+A311D
M731+T89K+C281 Y+T293K+G300S+R307T+A311 D
T89K+C281Y+T293K+G300S+A311D
T89K+C281Y+A311D
M73H+T89K+T213A+C281Y+A311D
T89K+R249C+C281 Y+R306?+A311 D
T89K+C281Y+R306?+A311D
and whereby the mutations are introduced in SEQ ID NO 1.

5. A polynucleotide encoding the mutant expandase of anyone of the preceding claims.

6. An expression vector or cassette comprising the polynucleotide of claim 5.

7. A host cell transformed with the polynucleotide of claim 5 or the vector or cassette of claim 6.

8. A method of producing the mutant expandase of claims 1-4 comprising cultivating a host cell according to claim 7 under conditions conducive to the production of the mutant expandase and, optionally, recovering the polypeptide.

9. A method of producing a β-lactam compound of interest comprising cultivating a host cell according to claim 7 under conditions conducive to the production of the β-lactam compound and, optionally, recovering the β-lactam compound.

10. A method according to claim 9, further comprising N-deacylating the β-lactam compound to produce an N-deacylated β-lactam compound.

11. Use of the mutant expandase polypeptide according to claim 1 to 4 in the expansion of the penam ring into a ceph-3-em ring.

12. Use of the mutant expandase polypeptide according to claim 1 to 4 in the expansion of the penam ring of adipyl-6-APA into a ceph-3-em ring.

13. Use according to claim 11 in the production of adipyl-7-ADCA, adipyl-7-ADAC or adipyl-7-ACA.

14. An adipyl-6-APA producing microorganism transformed with a DNA fragment encoding an altered expandase polypeptide according to claim 1 to 4.

15. Method for the fermentative production of adipyl-7-ADCA comprising
(a) Culturing a microorganism capable of producing adipyl-6-APA and transformed with a DNA fragment encoding an altered expandase according to claim 1 to 4 under adipyl-6-APA-producing conditions
(b) *In situ* converting the adipyl-6-APA into adipyl-7-ADCA using the altered expandase;and
(c) Isolating the desired adipyl-7-ADCA.

16. Method for the production of 7-ADCA comprising
(a) Culturing a microorganism capable of producing adipyl-6-APA and transformed with a DNA fragment encoding an altered expandase according to claim 1 to 4 under adipyl-6-APA-producing conditions
(b) *In situ* converting the adipyl-6-APA into adipyl-7-ADCA using the altered expandase;
(c) Isolating the desired adipyl-7-ADCA.
(d) Removal of the adipyl side chain to yield 7-ADCA; and
(e) Recovery of 7-ADCA.

## Patentansprüche

1. Mutierte Expandase, bei der es sich um eine Variante eines Modellpolypeptids mit Expandase-Aktivität handelt, wobei Threonin in Aminosäureposition 89 in dem Modellpeptid unter Verwendung der Aminosäurepositionsnummerierung der Aminosäuresequenz des Expandase-Enzyms, wie sie in SEQ ID NO: 1 dargestellt ist, durch Lysin ersetzt ist und wobei das Modellpolypeptid mit Expandase-Aktivität die Aminosäuresequenz, wie sie in SEQ ID NO: 1 dargestellt ist, oder eine Aminosäuresequenz mit einer Identität von wenigstens 70% mit SEQ ID NO: 1 aufweist.

2. Mutierte Expandase gemäß Anspruch 1, wobei die mutierte Expandase in Aminosäureposition 89 modifiziert ist, wie in Anspruch 1 beschrieben, und in wenigstens einer aus der aus Position 2, 18, 59, 73, 74, 90, 99, 101, 105, 112, 113, 155, 170, 177, 209, 213, 217, 244, 249, 251, 277, 278, 280, 281, 284, 293, 300, 307 und 311 unter Verwendung der Aminosäurepositionsnummerierung der von dem cefE-Gen aus *Streptomyces clavuligerus* codierten Aminosäuresequenz des Expandase-Enzyms (SEQ ID NO: 1) bestehenden Gruppe 1 ausgewählten Aminosäureposition modifiziert ist.

3. Mutierte Expandase gemäß Anspruch 1, wobei die mutierte Expandase in Aminosäureposition 89 modifiziert ist, wie in Anspruch 1 beschrieben, und in wenigstens einer aus der aus Position 2, 18, 59, 90, 99, 101, 105, 112, 113, 170, 177, 209, 213, 217, 249, 251, 278, 280, 284 und 293 bestehenden Gruppe 2 ausgewählten Aminosäureposition, gegebenenfalls in Kombination mit wenigstens einer aus der Gruppe 1 ausgewählten Aminosäureposition, die nicht der Gruppe 2 angehört, modifiziert ist.

4. Mutierte Expandase gemäß Anspruch 1, die aus der aus
T89K+C281Y+T293K
T89K+C281Y
M73H+T89K+C281Y+A311D
T89K+Y217H+C281Y+A311D
T89K+H244R+C281Y+A311D
T89K+C281Y+R307T+A311D
T89K+C281Y+R307T+A311D
M73I+T89K+C281Y+T293K+G300S+R307T+A311D
T89K+C281Y+T293K+G300S+A311D
T89K+C281Y+A311D
M73H+T89K+T213A+C281Y+A311D
T89K+R249C+C281Y+R306?+A311D
T89K+C281Y+R306?+A311D
bestehenden Gruppe ausgewählt ist, und wobei die Mutationen in SEQ ID NO 1 eingeführt werden.

5. Polynukleotid, codierend für die mutierte Expandase nach einem der vorhergehenden Ansprüche.

6. Expressionsvektor oder -kassette, umfassend das Polynukleotid nach Anspruch 5.

7. Wirtszelle, transformiert mit dem Polynukleotid nach Anspruch 5 oder dem Vektor bzw. der Kassette nach Anspruch 6.

8. Verfahren zur Herstellung der mutierten Expandase nach Anspruch 1-4, bei dem man eine Wirtszelle gemäß Anspruch 7 unter Bedingungen, die der Produktion der mutierten Expandase förderlich sind, kultiviert und gegebenenfalls das Polypeptid gewinnt.

9. Verfahren zur Herstellung einer interessierenden β-Lactam-Verbindung, bei dem man eine Wirtszelle gemäß Anspruch 7 unter Bedingungen, die der Produktion der β-Lactam-Verbindung förderlich sind, kultiviert und gegebenenfalls die β-Lactam-Verbindung gewinnt.

10. Verfahren gemäß Anspruch 9, bei dem man ferner die β-Lactam-Verbindung N-deacyliert, so dass eine N-deacylierte β-Lactam-Verbindung entsteht.

11. Verwendung des mutierten Expandase-Polypeptids gemäß Anspruch 1 bis 4 bei der Erweiterung des Penam-Rings zu einem Ceph-3-em-Ring.

12. Verwendung des mutierten Expandase-Polypeptids gemäß Anspruch 1 bis 4 bei der Erweiterung des Penam-Rings von Adipyl-6-APA zu einem Ceph-3-em-Ring.

13. Verwendung gemäß Anspruch 11 bei der Herstellung von Adipyl-7-ADCA, Adipyl-7-ADAC oder Adipyl-7-ACA.

14. Adipyl-6-APA produzierender Mikroorganismus, transformiert mit einem für ein verändertes Expandase-Polypeptid gemäß Anspruch 1 bis 4 codierenden DNA-Fragment.

15. Verfahren zur fermentativen Herstellung von Adipyl-7-ADCA, wobei man
(a) einen zur Produktion von Adipyl-6-APA fähigen und mit einem für eine veränderte Expandase gemäß Anspruch 1 bis 4 codierenden DNA-Fragment transformierten Mikroorganismus unter Adipyl-6-APA produzierenden Bedingungen kultiviert,
(b) die Adipyl-6-APA in situ unter Verwendung der veränderten Expandase in Adipyl-7-ADCA umwandelt und
(c) die gewünschte Adipyl-7-ADCA isoliert.

16. Verfahren zur Herstellung von 7-ADCA, wobei man
(a) einen zur Produktion von Adipyl-6-APA fähigen und mit einem für eine veränderte Expandase gemäß Anspruch 1 bis 4 codierenden DNA-Fragment transformierten Mikroorganismus unter Adipyl-6-APA produzierenden Bedingungen kultiviert,
(b) die Adipyl-6-APA in situ unter Verwendung der veränderten Expandase in Adipyl-7-ADCA umwandelt,
(c) die gewünschte Adipyl-7-ADCA isoliert,
(d) die Adipyl-Seitenkette unter Erhalt von 7-ADCA abtrennt und
(e) 7-ADCA gewinnt.

## Revendications

1. Expandase mutante qui est un variant d'un polypeptide modèle ayant une activité d'expandase, moyennant quoi une thréonine en une position d'acide aminé 89 dans le peptide modèle a été remplacée par une lysine, en utilisant la numérotation des positions d'acides aminés de la séquence d'acides aminés de l'enzyme expandase telle que décrite dans la SEQ ID n° : 1, et moyennant quoi le polypeptide modèle avec l'activité d'expandase a la séquence d'acides aminés telle que décrite dans la SEQ ID n° : 1 ou une séquence d'acides aminés ayant une identité d'au moins 70% avec la SEQ ID n° : 1.

2. Expandase mutante, selon la revendication 1, moyennant quoi l'expandase mutante a été modifiée en une position d'acide aminé 89, tel que décrit dans la revendication 1, et a été modifiée au moins en une position d'acide aminé choisie dans le groupe 1 constitué par les positions 2, 18, 59, 73, 74, 90, 99, 101, 105, 112, 113, 155, 170, 177, 209, 213, 217, 244, 249, 251, 277, 278, 280, 281, 284, 293, 300, 307 et 311, en utilisant la numérotation des positions d'acides aminés de la séquence d'acides aminés de l'enzyme expandase codée par le gène cefE de *Streptomyces clavuligerus* (SEQ ID n° : 1).

3. Expandase mutante, selon la revendication 1, moyennant quoi l'expandase mutante a été modifiée au niveau de la position d'acide aminé 89, tel que décrit à la revendication 1, et a été modifiée au moins en une position d'acide aminé choisie dans le groupe 2 constitué par les positions 2, 18, 59, 90, 99, 101, 105, 112, 113, 170, 177, 209, 213, 217, 249, 251, 278, 280, 284 et 293, étant facultativement en combinaison avec au moins une position d'acide aminé choisie dans le groupe 1 et qui n'appartient pas au groupe 2.

4. Expandase mutante, selon la revendication 1, et qui est choisie dans le groupe constitué par :
**T69K+C281Y+T293K**
**T89K+C281Y**
**M73H+T89K+C281Y+AS11D**
**T89K+Y217H+C281Y+A311D**
**T89K+H244R+C281Y+A311D**
**T89K+C281Y+R307T+A311D**
**T89K+C281Y+R307T+A311D**
**M73I+T89K+C281Y+T293K+-G300S+R307T+A311D**
**T89K+C281Y+T293K+G300S+A311D**
**T89K+C281Y+A311D**
**M73H+T89K+T213A+C281Y+A311D**
**T89K+R249C+C281Y+R306+A311D**
**T89K+C281Y+R306?+A311D**
et dans laquelle les mutations sont introduites dans la SEQ ID n° : 1.

5. Polynucléotide codant pour l'expandase mutante selon l'une quelconque des revendications précédentes.

6. Vecteur ou cassette d'expression comprenant le polynucléotide selon la revendication 5.

7. Cellule hôte transformée avec le polynucléotide, selon la revendication 5, ou bien le vecteur ou la cassette selon la revendication 6.

8. Procédé de production de l'expandase mutante, selon les revendications 1 à 4, comprenant les étapes consistant à cultiver une cellule hôte, selon la revendication 7, dans des conditions conduisant à la production de l'expandase mutante et, en option, récupérer le polypeptide.

9. Procédé de production d'un composé d'intérêt de β-lactamine comprenant les étapes consistant à cultiver une cellule hôte, selon la revendication 7, dans des conditions conduisant à la production du composé de β-lactamine et, en option, récupérer le composé de β-lactamine.

10. Procédé, selon la revendication 9, comprenant en outre l'étape de N-désacylation du composé de β-lactamine pour produire un composé de β-lactamine N-désacylée.

11. Utilisation du polypeptide d'expandase mutante, selon les revendications 1 à 4, dans l'expansion de l'anneau péname en un anneau céph-3-em.

12. Utilisation du polypeptide d'expandase mutante, selon les revendications 1 à 4, dans l'expansion de l'anneau péname de l'adipyl-6-APA en un anneau céph-3-em.

13. Utilisation, selon la revendication 11, dans la production d'adipyl-7-ADCA, d'adipyl-7-ADAC ou d'adipyl-7-ACA.

14. Microorganisme producteur d'adipyl-6-APA transformé avec un fragment d'ADN codant pour un polypeptide d'expandase altérée selon les revendications 1 à 4.

15. Procédé pour la production fermentative d'adipyl-7-ADCA, comprenant les étapes consistant à :
(a) cultiver un microorganisme étant capable de produire de l'adipyl-6-APA et transformé avec un fragment d'ADN codant pour une expandase altérée, selon les revendications 1 à 4, dans des conditions de production de l'adipyl-6-APA ;
(b) convertir *in situ* l'adipyl-6-APA en adipyl-7-ADCA, en utilisant l'expandase altérée ; et
(c) isoler l'adipyl-7-ADCA souhaité.

16. Procédé pour la production de 7-ADCA comprenant les étapes consistant à :
(a) cultiver un microorganisme étant capable de produire de l'adipyl-6-APA et transformé avec un fragment d'ADN codant pour une expandase altérée, selon les revendications 1 à 4, dans des conditions de production de l'adipyl-6-APA ;
(b) convertir *in situ* l'adipyl-6-APA en adipyl-7-ADCA, en utilisant l'expandase altérée ;
(c) isoler l'adipyl-7-ADCA souhaité ;
(d) retirer la chaîne latérale de l'adipyle pour produire le 7-ADCA ; et
(e) récupérer le 7-ADCA.
